(19) Europäisches Patentamt
European Patent Office
Office européen des brevets

(11) EP 0 946 526 B1

(12) **EUROPÄISCHE PATENTSCHRIFT**

(45) Veröffentlichungstag und Bekanntmachung des Hinweises auf die Patenterteilung:
**29.10.2003 Patentblatt 2003/44**

(21) Anmeldenummer: **97952805.6**

(22) Anmeldetag: **26.11.1997**

(51) Int Cl.[7]: **C07D 257/02**, A61K 49/00

(86) Internationale Anmeldenummer:
**PCT/EP97/06593**

(87) Internationale Veröffentlichungsnummer:
**WO 98/024774 (11.06.1998 Gazette 1998/23)**

(54) **MACROCYCLISCHE METALLKOMPLEXCARBONSÄUREN, IHRE VERWENDUNG SOWIE VERFAHREN ZU IHRER HERSTELLUNG**

MACROCYCLIC METAL COMPLEX CARBOXYLIC ACIDS, USE AND METHOD FOR THE PRODUCTION THEREOF

ACIDES CARBOXYLIQUES A COMPLEXES METALLIFERES MACROCYCLIQUES, LEUR UTILISATION ET PROCEDE PERMETTANT DE LES PREPARER

(84) Benannte Vertragsstaaten:
**AT BE CH DE DK ES FI FR GB GR IE IT LI LU MC NL PT SE**

(30) Priorität: **04.12.1996 DE 19652387**

(43) Veröffentlichungstag der Anmeldung:
**06.10.1999 Patentblatt 1999/40**

(73) Patentinhaber: **SCHERING AKTIENGESELLSCHAFT**
**13342 Berlin (DE)**

(72) Erfinder:
- **PLATZEK, Johannes**
  **D-12621 Berlin (DE)**
- **SCHMITT-WILLICH, Heribert**
  **D-12161 Berlin (DE)**
- **RADÜCHEL, Bernd**
  **D-13465 Berlin (DE)**

(56) Entgegenhaltungen:
**EP-A- 0 430 863**
**EP-A- 0 448 191**
**WO-A-96/01655**
**WO-A-97/02051**
**DE-A- 4 344 460**
**DE-A- 19 525 924**
**DE-A- 19 549 286**

Bemerkungen:
Die Akte enthält technische Angaben, die nach dem Eingang der Anmeldung eingereicht wurden und die nicht in dieser Patentschrift enthalten sind.

EP 0 946 526 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft den in den Patentansprüchen gekennzeichneten Gegenstand, das heißt neue makrocyclische Metallkomplexcarbonsäuren, ihre Verwendung sowie Verfahren zu ihrer Herstellung.

Zum Stand der Technik wird auf den folgenden Seiten 1-23 aus der am 23.01.97 publizierten Anmeldung WO-A-97/02051 zitiert:

Die zur Zeit klinisch eingesetzten Kontrastmittel für die modernen bildgebenden Verfahren Kernspintomographie (MRI) und Computertomographie (CT) [Magnevist ®, Pro Hance ®, Ultravist® und Omniscan ®] verteilen sich im gesamten extrazellulären Raum des Körpers (Intravasalraum und Interstitium). Dieser Verteilungsraum umfaßt etwa 20 % des Korpervolumens.

Extrazelluläre MRI-Kontrastmittel sind klinisch zuerst erfolgreich bei der Diagnostik von zerebralen und spinalen Krankheitsprozessen eingesetzt worden, da sich hier eine ganz besondere Situation hinsichtlich des regionalen Verteilungsraumes ergibt. Im Gehirn und im Rückenmark können extrazelluläre Kontrastmittel im gesunden Gewebe aufgrund der Blut-Hirn-Schranke nicht den Intravasalraum verlassen. Bei krankhaften Prozessen mit Störung der Blut-Hirn-Schranke (z.B. maligne Tumoren, Entzündungen, demyelini-sierende Erkrankungen etc.) entstehen innerhalb des Hirns dann Regionen mit erhöhter Blutgefäß-Durchlässigkeit (Permeabilität) für diese extrazellulären Kontrastmittel (Schmiedl et al., MRI of blood-brain barrier permeability in astrocytic gliomas: application of small and large molecular weight contrast media, Magn. Reson. Med. 22: 288, 1991). Durch das Ausnutzen dieser Störung der Gefäßpermeabilität kann erkranktes Gewebe mit hohem Kontrast gegenüber dem gesunden Gewebe erkannt werden.

Außerhalb des Gehirns und des Rückenmarkes gibt es allerdings eine solche Permeabilitätsbarriere für die oben genannten Kontrastmittel nicht (Canty et al., First-pass entry of nonionic contrast agent into the myocardial extravascular space. Effects on radiographic estimate of transit time and blood volume. Circulation 84: 2071, 1991). Damit ist die Anreicherung des Kontrastmittels nicht mehr abhängig von der Gefäßpermeabilität, sondern nur noch von der Größe des extrazellulären Raumes im entsprechenden Gewebe. Eine Abgrenzung der Gefäße gegenüber dem umliegenden interstitiellen Raum bei Anwendung dieser Kontrastmittel ist nicht möglich.

Besonders für die Darstellung von Gefäßen wäre ein Kontrastmittel wünschenswert, das sich ausschließlich im vasalen Raum (Gefäßraum) verteilt. Ein solches blood-pool-agent soll es ermöglichen, mit Hilfe der Kernspintomographie gut durchblutetes von schlecht durchblutetem Gewebe abzugrenzen und somit eine Ischämie zu diagnostizieren. Auch infarziertes Gewebe ließe sich aufgrund seiner Anämie vom umliegenden gesunden oder ischämischen Gewebe abgrenzen, wenn ein vasales Kontrastmittel angewandt wird. Dies ist von besonderer Bedeutung, wenn es z.B. darum geht, einen Herzinfarkt von einer Ischämie zu unterscheiden.

Bisher müssen sich die meisten der Patienten, bei denen Verdacht auf eine kardiovaskuläre Erkrankung besteht (diese Erkrankung ist die häufigste Todesursache in den westlichen Industrieländern), invasiven diagnostischen Untersuchungen unterziehen. In der Angiographie wird zur Zeit vor allem die Röntgen-Diagnostik mit Hilfe von jodhaltigen Kontrastmitteln angewandt. Diese Untersuchungen sind mit verschiedenen Nachteilen behaftet: sie sind mit dem Risiko der Strahlenbelastung verbunden, sowie mit Unannehmlichkeiten und Belastungen, die vor allem daher kommen, daß die jodhaltigen Kontrastmittel, verglichen mit NMR-Kontrastmitteln, in sehr viel höherer Konzentration angewandt werden müssen.

Es besteht daher ein Bedarf an NMR-Kontrastmitteln, die den vasalen Raum markieren können (blood-pool-agent). Diese Verbindungen sollen sich durch eine gute Vertraglich-keit und durch eine hohe Wirksamkeit (hohe Steigerung der Signalintensität bei MRI) auszeichnen.

Der Ansatz, zumindest einen Teil dieser Probleme durch Verwendung von Komplexen, die an Makro- oder Biomoleküle gebunden sind, zu lösen, war bisher nur sehr begrenzt erfolgreich.

So ist beispielsweise die Anzahl paramagnetischer Zentren in den Komplexen, die in den Europäischen Patentanmeldungen Nr. 0 088 695 und Nr. 0 150 844 beschrieben sind, für eine zufriedenstellende Bildgebung nicht ausreichend.

Erhöht man die Anzahl der benötigten Metallionen durch mehrfache Einführung komplexierender Einheiten in ein makromolekulares Biomolekül, so ist das mit einer nicht tolerierbaren Beeinträchtigung der Affinität und/oder Spezifizität dieses Biomoleküls verbunden [J. Nucl. Med. 24, 1158 (1983)].

Makromoleküle können generell als Kontrastmittel für die Angiographie geeignet sein. Albumin-GdDTPA (Radiology 1987; 162: 205) z.B. zeigt jedoch 24 Stunden nach intravenöser Injektion bei der Ratte eine Anreicherung im Lebergewebe, die fast 30 % der Dosis ausmacht. Außerdem werden in 24 Stunden nur 20 % der Dosis eliminiert.

Das Makromolekül Polylysin-GdDTPA (Europäische Patentanmeldung, Publikations-Nr. 0 233 619) erwies sich ebenfalls geeignet als blood-pool-agent. Diese Verbindung besteht jedoch herstellungsbedingt aus einem Gemisch von Molekülen verschiedener Größe. Bei Ausscheidungsversuchen bei der Ratte konnte gezeigt werden, daß dieses Makromolekül unverändert durch glomeruläre Filtration über die Niere ausgeschieden wird. Synthesebedingt kann Polylysin-GdDTPA aber auch Makromoleküle enthalten, die so groß sind, daß sie bei der glomerulären Filtration die Kapillaren der Niere nicht passieren können und somit im Körper zurückbleiben.

Auch makromolekulare Kontrastmittel auf der Basis von Kohlenhydraten, z.B. Dextran, sind beschrieben worden (Europäische Patentanmeldung, Publikations-Nr. 0 326 226). Der Nachteil dieser Verbindungen liegt darin, daß diese in der Regel nur ca. 5 % des signalverstärkenden paramagnetischen Kations tragen.

Die in der Europäischen Patentanmeldung Nr. 0 430 863 beschriebenen Polymere stellen bereits einen Schritt auf dem Wege zu blood-pool-agents dar, da sie nicht mehr die für die vorher erwähnten Polymere charakteristische Heterogenität bezüglich Größe und Molmasse aufweisen. Sie lassen jedoch immer noch Wünsche im Hinblick auf vollständige Ausscheidung, Verträglichkeit und/oder Wirksamkeit offen.

Wie in der PCT/EP 96/02671 beschrieben, wurde gefunden, daß sich Komplexe, die aus stickstoffhaltigen, mit komplexbildenden Liganden versehenen Kaskaden-Polymeren, mindestens 16 Ionen eines Elements der Ordnungszahlen 20 - 29, 39, 42, 44 oder 57 - 83 sowie gegebenenfalls Kationen anorganischer und/oder organischer Basen, Aminosäuren oder Aminosäureamide bestehen und gegebenenfalls acylierte Aminogruppen enthalten, überraschenderweise hervorragend zur Herstellung von NMR- und Röntgen-Diagnostika ohne die genannten Nachteile aufzuweisen, eignen.

Die in der angegebenen Patentanmeldung beschriebenen komplexbildenden Kaskaden-Polymere lassen sich durch die allgemeine Formel I beschreiben

$$A\text{-}\{X\text{-}[Y\text{-}(Z\text{-}\langle W\text{-}K_w\rangle_z)_y]_x\}_a \qquad \text{(I)},$$

worin

| | |
|---|---|
| A | für einen stickstoffhaltigen Kaskadenkern der Basismultiplizität a, |
| X und Y | unabhängig voneinander für eine direkte Bindung oder eine Kaskadenreproduktionseinheit der Reproduktionsmultiplizität x bzw. y, |
| Z und W | unabhängig voneinander für eine Kaskadenreproduktionseinheit der Reproduktionsmultiplizität z bzw. w, |
| K | für den Rest eines Komplexbildners, |
| a | für die Ziffern 2 bis 12, |
| x, y, z und w | unabhängig voneinander für die Ziffern 1 bis 4 stehen, |

mit der Maßgabe, daß mindestens zwei Reproduktionseinheiten unterschiedlich sind und daß für das Produkt der Multiplizitäten gilt

$$16 \le a \cdot x \cdot y \cdot z \cdot w \le 64.$$

Als Kaskadenkern A sind geeignet:
Stickstoffatom,

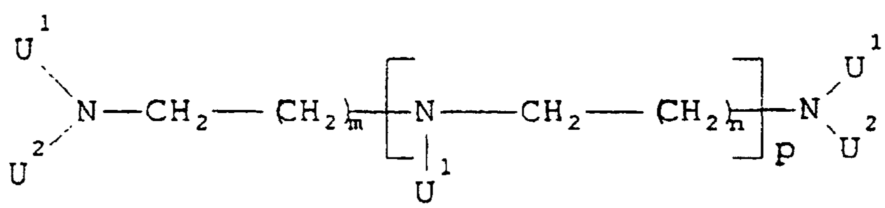

,

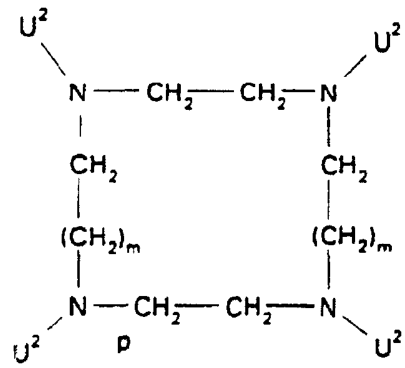

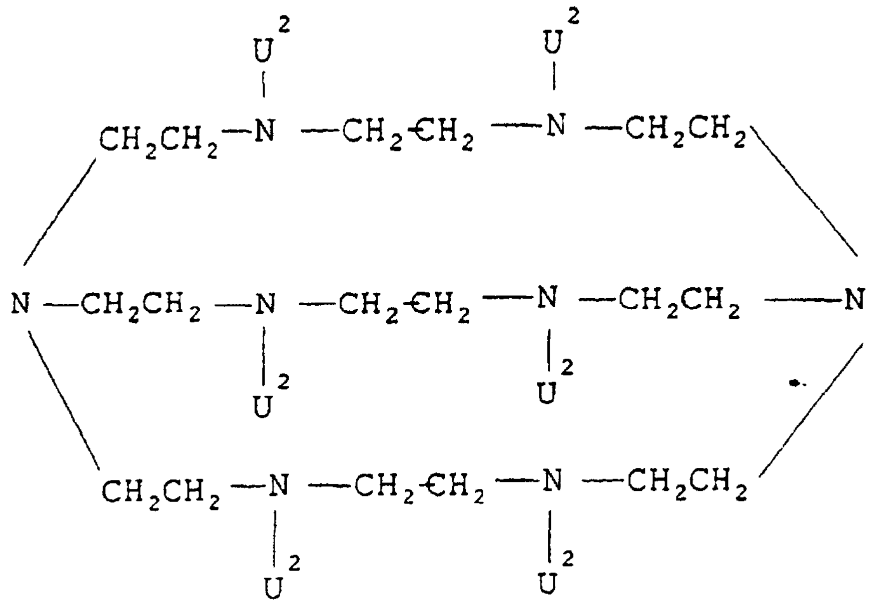

,

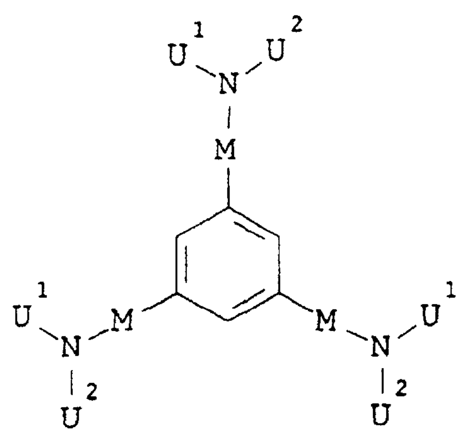

,

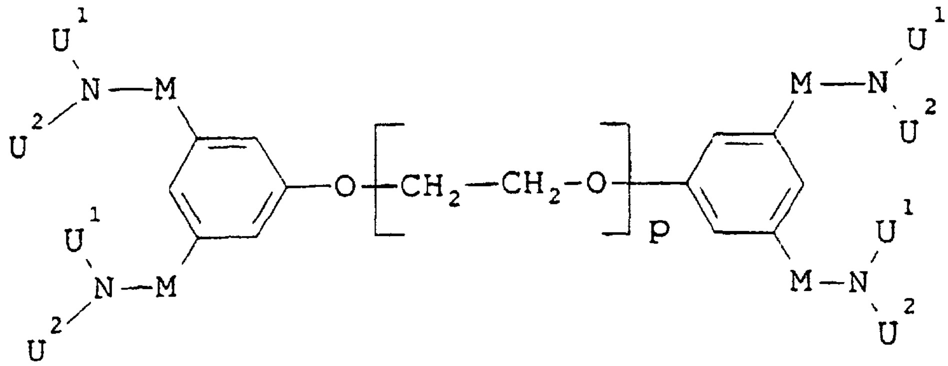

,

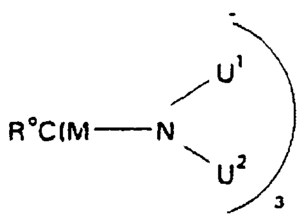

,

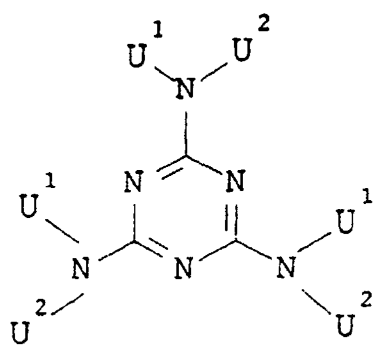

,

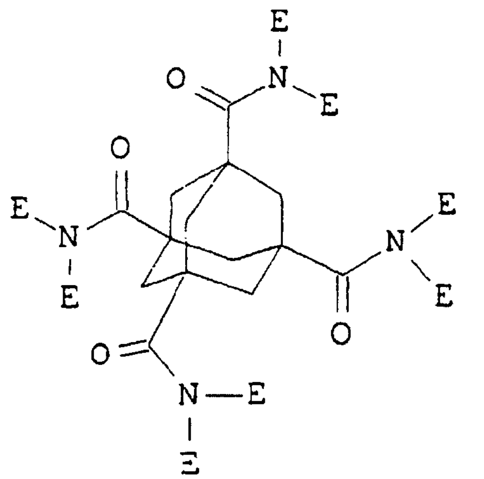

,

worin

- m und n für die Ziffern 1 bis 10,
- p für die Ziffern 0 bis 10,
- $U^1$ für $Q^1$ oder E,
- $U^2$ für $Q^2$ oder E mit
- E in der Bedeutung der Gruppe

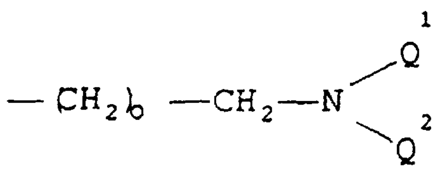

,

wobei

- o für die Ziffern 1 bis 6,
- $Q^1$ für ein Wasserstoffatom oder $Q^2$ und
- $Q^2$ für eine direkte Bindung

- M für eine $C_1$-$C_{10}$-Alkylenkette, die gegebenenfalls durch 1 bis 3 Sauerstoffatome unterbrochen ist und/oder gegebenenfalls mit 1 bis 2 Oxogruppen substituiert ist,
- $R^o$ für einen verzweigten oder unverzweigten $C_1$-$C_{10}$-Alkylrest, eine Nitro-, Amino-, Carbonsäuregruppe oder für

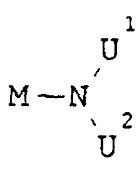

stehen,

wobei die Anzahl $Q^2$ der Basismultiplizität a entspricht.

Den einfachsten Fall eines Kaskadenkerns stellt das Stickstoffatom dar, dessen drei Bindungen (Basismultiplizität a = 3) in einer ersten "inneren Schicht" (Generation 1) von drei Reproduktionseinheiten X bzw. Y (wenn X für eine direkte Bindung steht) bzw. Z (wenn X und Y jeweils für eine direkte Bindung stehen) besetzt sind; anders formuliert: die drei Wasserstoffatome des zugrundeliegenden Kaskadenstarters Ammoniak $A(H)_a = NH_3$ sind durch drei

Reproduktionseinheiten X bzw. Y bzw. Z substituiert worden. Die im Kaskadenkem A enthaltene Anzahl $Q^2$ gibt dabei die Basismulitplizität a wieder.

Die Reproduktionseinheiten X, Y, Z und W enthalten $-NQ^1Q^2$-Gruppen, worin $Q^1$ ein Wasserstoffatom oder $Q^2$ und $Q^2$ eine direkte Bindung bedeuten. Die in der jeweiligen Reproduktionseinheit (z.B. X) enthaltene Anzahl $Q^2$ entspricht der Reproduktionsmultiplizität dieser Einheit (z.B. x im Falle von X). Das Produkt aller Multiplizitäten $a \cdot x \cdot y \cdot z \cdot w$ gibt die Anzahl der im Kaskadenpolymeren gebundenen Komplexbildner-Reste K an. Die erfindungsgemäßen Polymere enthalten mindestens 16 und höchstens 64 Reste K im Molekül, die jeweils ein bis maximal drei (im Falle von zweiwertigen Ionen), vorzugs-weise ein Ion, eines Elements der oben genannten Ordnungszahlen binden können.

Die letzte Generation, d.h. die an die Komplexbildner-Reste K gebundene Reproduktionseinheit W ist über NH-Gruppen ($-NQ^1Q^2$ mit Q1 in der Bedeutung eines Wasserstoff-atoms und $Q^2$ = direkte Bindung) an K gebunden, während die vorangehenden Reproduktionseinheiten sowohl über $NHQ^2$-Gruppen (z.B. durch Acylierungsreaktionen) als auch über $NQ^2Q^2$-Gruppen (z.B. durch Alkylierungsreaktionen) miteinander verknüpft sein können.

Die Kaskaden-Polymer-Komplexe weisen maximal 10 Generationen auf (d.h. es können auch mehr als jeweils nur eine der Reproduktionseinheiten X, Y und Z im Molekül vorhanden sein), vorzugsweise jedoch 2 bis 4 Generationen, wobei mindestens zwei der Reproduktionseinheiten im Molekül unterschiedlich sind.

Als bevorzugte Kaskadenkerne A sind diejenigen angeführt, die unter die oben genannten allgemeinen Formeln fallen, wenn

m für die Ziffern 1 - 3, besonders bevorzugt für die Ziffer 1,
n für die Ziffern 1 - 3, besonders bevorzugt für die Ziffer 1,
p für die Ziffern 0 - 3, besonders bevorzugt für die Ziffer 1,
o für die Ziffer 1,
M für eine $-CH_2-$, -CO- oder $-CH_2CO$-Gruppe und
$R^o$ für eine $-CH_2NU^1U^2-$, $CH_3$- oder $NO_2$-Gruppe

steht.

Als weitere bevorzugte Kaskadenstarter A(H)a sind beispielhaft aufgeführt:
(In der Klammer wird die Basismultiplizität a angegeben für den Fall der zum Aufbau der nächsten Generation dienenden nachfolgenden Mono- bzw. Disubstitution)

| | |
|---|---|
| Tris(aminoethyl)amin | (a = 6 bzw. 3); |
| Tris(aminopropyl)amin | (a = 6 bzw. 3); |
| Diethylentriamin | (a = 5 bzw. 3); |
| Triethylentetramin | (a = 6 bzw. 4); |
| Tetraethylenpentamin | (a = 7 bzw. 5); |
| 1,3,5-Tris(aminomethyl)benzol | (a = 6 bzw. 3); |
| Trimesinsäuretriamid | (a = 6 bzw. 3); |
| 1,4,7-Triazacyclononan | (a = 3); |
| 1,4,7,10-Tetraazacyclododecan | (a = 4); |
| 1,4,7,10,13-Pentaazacyclopentadecan | (a = 5); |
| 1,4,8,11-Tetraazacyclotetradecan | (a = 4); |
| 1,4,7,10,13,16-Hexaazacyclooctadecan | (a = 6); |
| 1,4,7,10,13,16,19,22,25,28-Decaazacyclotriacontan | (a = 10); |
| Tetrakis(aminomethyl)methan | (a = 8 bzw. 4); |
| 1,1,1-Tris(aminomethyl)ethan | (a = 6 bzw. 3); |
| Tris(aminopropyl)-nitromethan | (a = 6 bzw. 3); |
| 2,4,6-Triamino-1,3,5-triazin | (a = 6 bzw. 3); |
| 1,3,5,7-Adamantantetracarbonsaureamid | (a= 8 bzw. 4); |
| 3,3',5,5'-Diphenylether-tetracarbonsäureamid | (a = 8 bzw. 4); |
| 1,2-Bis[Phenoxyethan]-3',3",5',5"-tetracarbonsäureamid | (a = 8 bzw. 4); |
| 1,4,7,10,13,16,21,24-Octaazabicyclo[8.8.8]hexacosan | (a = 6). |

Es wird darauf hingewiesen, daß die Definition als Kaskadenkern A und damit die Trennung von Kaskadenkern und erster Reproduktionseinheit rein formal und damit unabhängig von dem tatsächlichen synthetischen Aufbau der gewünschten Kaskaden-Polymer-Komplexe gewählt werden kann. So kann man z.B. das in Beispiel 4 verwendete

Tris(aminoethyl)-amin sowohl selbst als Kaskadenkern A (vergleiche die erste für A angegebene allgemeine Formel mit m = n = p = 1, $U^1$ = E mit o in der Bedeutung der Ziffer 1 und $U^1 = U^2 = Q^2$) aber auch als Stickstoffatom (= Kaskadenkern A), das als erste Generation drei Reproduktionseinheiten

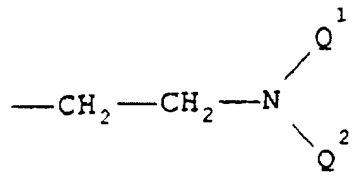

(vergleiche die Definition von E) aufweist, ansehen.

Die Kaskadenreproduktionseinheiten X, Y, Z und W werden unabhängig voneinander durch

E,

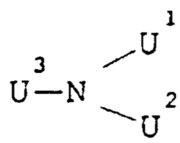

,

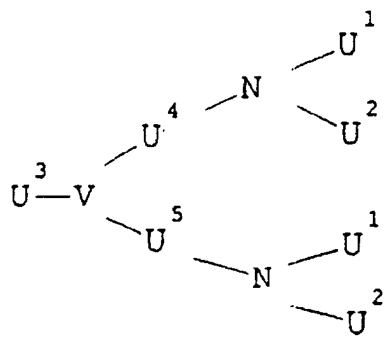

,

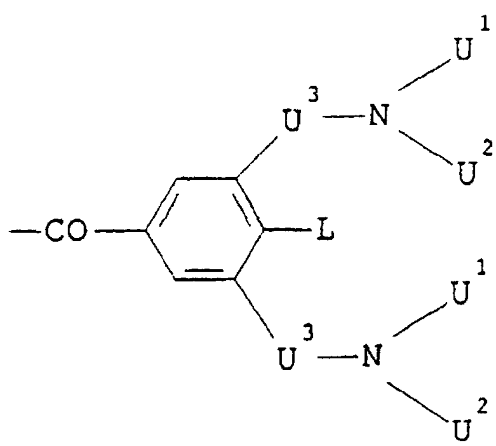

bestimmt,

worin

$U^1$ für $Q^1$ oder E,

$U^2$ für $Q^2$ oder E mit

E in der Bedeutung der Gruppe

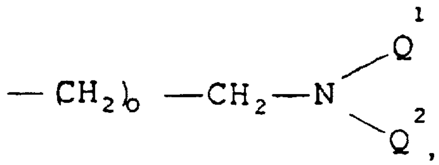

wobei

o für die Ziffern 1 bis 6,
$Q^1$ für ein Wasserstoffatom oder $Q^2$ ,
$Q^2$ für eine direkte Bindung,

$U^3$ für eine $C_1$-$C_{20}$-Alkylenkette, die gegebenenfalls durch 1 bis 10 Sauerstoffatome und/oder 1 bis 2 -$N(CO)_q$-$R^2$-, 1 bis 2 Phenylen- und/oder 1 bis 2 Phenylenoxyreste unterbrochen ist und/oder gegebenenfalls durch 1 bis 2 Oxo-, Thioxo-, Carboxy-, $C_1$-$C_5$-Alkylcarboxy-, $C_1$-$C_5$-Alkoxy-, Hydroxy-, $C_1$-$C_5$-alkylgruppen substituiert ist, wobei

q für die Ziffern 0 oder 1 und
$R^2$ für ein Wasserstoffatom, einen Methyl- oder einen Ethylrest, der gegebenenfalls mit 1 - 2 Hydroxy- oder 1 Carboxygruppe(n) substituiert ist,
L für ein Wasserstoffatom oder die Gruppe

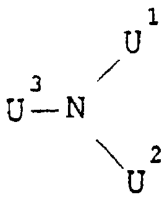

V für die Methingruppe

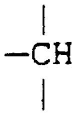

wenn gleichzeitig $U^4$ eine direkte Bindung oder die Gruppe M bedeutet und $U^5$ eine der Bedeutungen von $U^3$ besitzt oder
V für die Gruppe

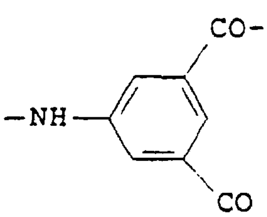

,

wenn gleichzeitig $U^4$ und $U^5$ identisch sind und die direkte Bindung oder die Gruppe M bedeuten, stehen.

Bevorzugte Kaskadenreproduktionseinheiten X, Y, Z und W sind diejenigen, bei denen in den oben genannten allgemeinen Formeln der Rest $U^3$ für -CO-, $-COCH_2OCH_2CO-$, $-COCH_2-$, $-CH_2CH_2-$, $-CONHC_6H_4-$, $-COCH_2CH_2CO-$, $-COCH_2-CH_2CH_2CO-$, $-COCH_2CH_2CH_2CH_2CO-$, der Rest $U^4$ für eine direkte Bindung, für $-CH_2CO-$, der Rest $U^5$ für eine direkte Bindung, für $-(CH_2)_4-$, $-CH_2CO-$, -CH(COOH)-, $CH_2OCH_2CH_2-$, $-CH_2C_6H_4-$, $CH_2-C_6H_4OCH_2CH_2-$, der Rest E für eine Gruppe

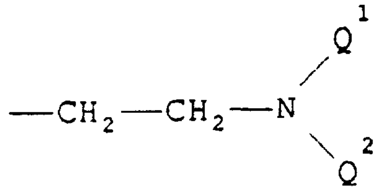

steht.

Als beispielhaft genannte Kaskadenreproduktionseinheiten X, Y, Z und W sind angeführt:
$-CH_2CH_2NH-$; $-CH_2CH_2N<$ ;
$-COCH(NH-)(CH_2)_4NH-$; $-COCH(N<)(CH_2)_4N<$ ;
$-COCH_2OCH_2CON(CH_2CH_2NH-)_2$; $-COCH_2OCH_2CON(CH_2CH_2N<)_2$ ;
$-COCH_2N(CH_2CH_2NH-)_2$; $-COCH_2N(CH_2CH_2N<)_2$;
$-COCH_2NH-$; $-COCH_2N<$ ;
$-COCH_2CH_2CON(CH_2CH_2NH-)_2$; $-COCH_2CH_2CON(CH_2CH_2N<)_2$ ;
$-COCH_2OCH_2CONH-C_6H_4-CH[CH_2CON(CH_2CH_2NH-)_2]_2$;
$-COCH_2OCH_2CONH-C_6H_4-CH[CH_2CON(CH_2CH_2N<)_2]_2$ ;
$-COCH_2CH_2CO-NH-C_6H_4-CH[CH_2CON(CH_2CH_2NH-)_2]_2$ ;
$-COCH_2CH_2CO-NH-C_6H_4-CH[CH_2CON(CH_2CH_2N<)_2]_2$;
$-CONH-C_6H_4-CH[CH_2CON(CH_2CH_2NH-)_2]_2$ ;
$-CONH-C_6H_4-CH[CH_2CON(CH_2CH_2N<)_2]_2$ ;
$-COCH(NH-)CH(COOH)NH-$; $-COCH(N<)CH(COOH)N<$ ;

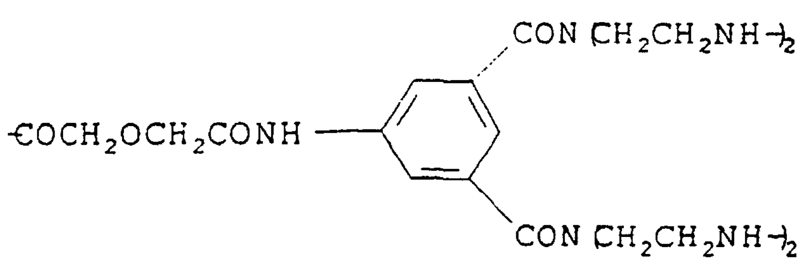

;

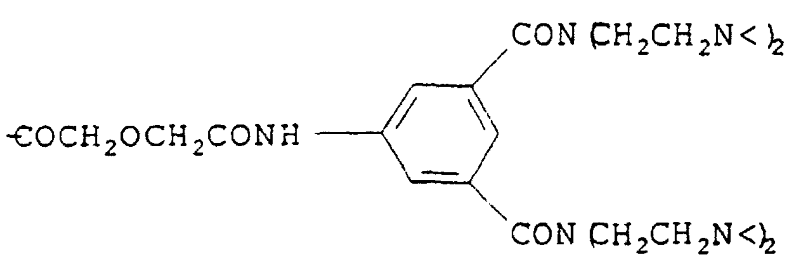

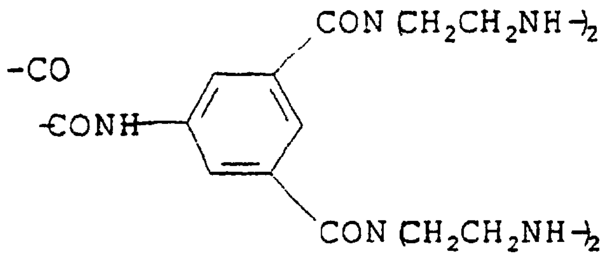

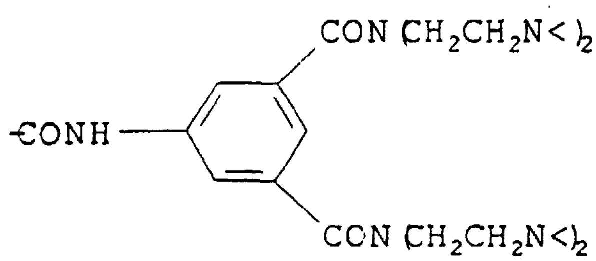

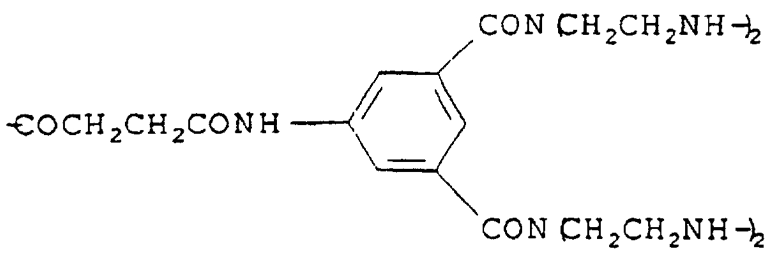

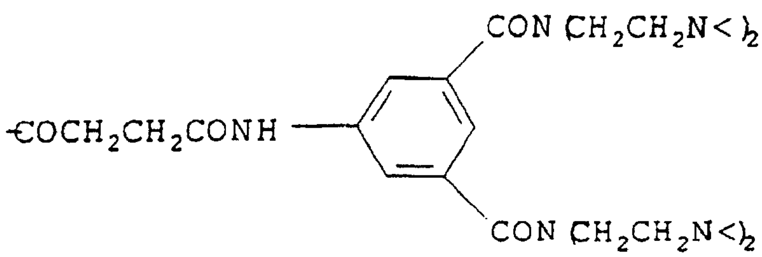

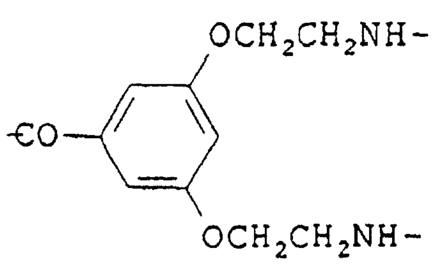

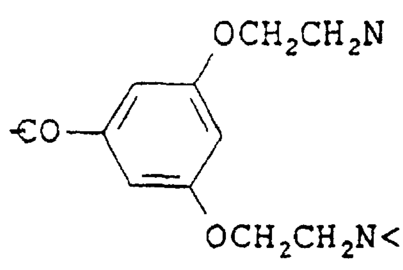

;

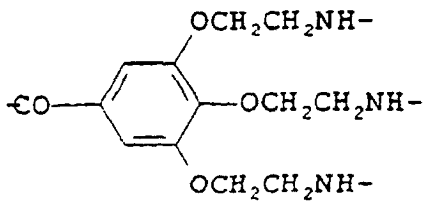

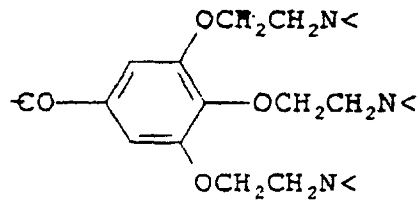

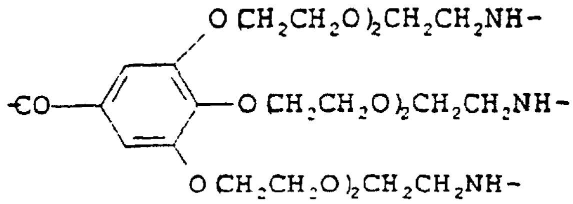

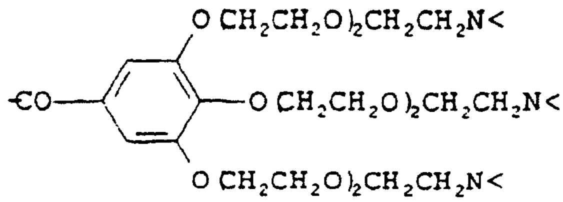

Der Komplexbildner-Rest K wird im Falle eines Macrocyclus' durch die allgemeine Formel IA beschrieben:

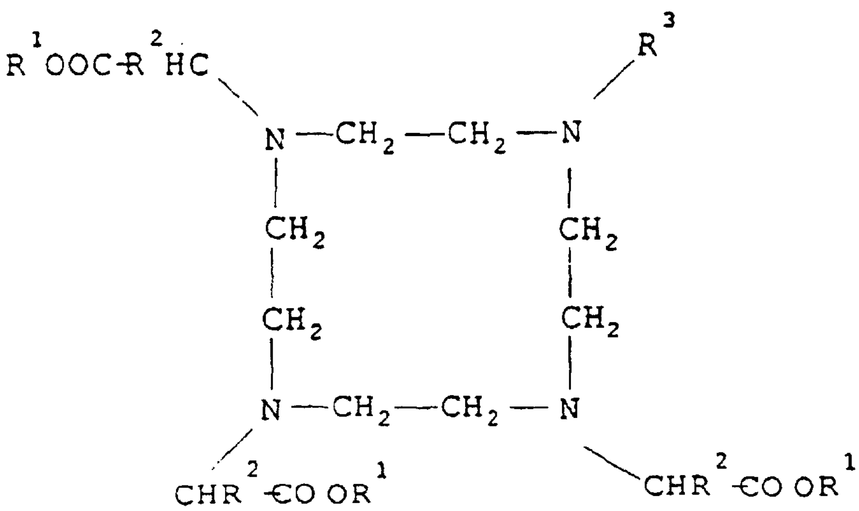

(IA),

worin

$R^1$ unabhängig voneinander für ein Wasserstoffatom oder ein Metallionenäquivalent der Ordnungszahlen 20 - 29, 39, 42 - 44 oder 57 - 83,

$R^2$ für ein Wasserstoffatom, einen Methyl- oder einen Ethylrest, der gegebenenfalls mit 1 - 2 Hydroxy- oder 1 Carboxygruppe(n) substituiert ist,

$R^3$ für eine

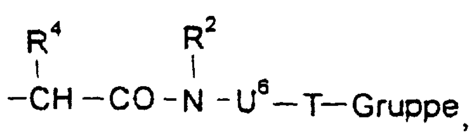

$R^4$ für eine geradkettige, verzweigte, gesättigte oder ungesättigte $C_1$-$C_{30}$-Alkylkette, die gegebenenfalls durch 1 - 10 Sauerstoffatome, 1 Phenylen-, 1 Phenylenoxygruppen unterbrochen und/oder gegebenenfalls durch 1 - 5 Hydroxy-, 1 - 3 Carboxy-, 1 Phenylgruppe(n) substituiert ist,

$U^6$ für eine gegebenenfalls 1 - 5 Imino-, 1 - 3 Phenylen-, 1 - 3 Phenylenoxy-, 1 - 3 Phenylenimino-, 1 - 5 Amid-, 1 - 2 Hydrazid-, 1 - 5 Carbonyl-, 1 - 5 Ethylenoxy-, 1 Harnstoff-, 1-Thioharnstoff-, 1 - 2 Carboxyalkyl imino-, 1 - 2 Estergruppen, 1 - 10 Sauerstoff-, 1 - 5 Schwefel- und/oder 1 - 5 Stickstoff-Atom(e) enthaltende und/oder gegebenenfalls durch 1 - 5 Hydroxy-, 1 - 2 Mercapto-, 1 - 5 Oxo-, 1 - 5 Thioxo-, 1 - 3 Carboxy-, 1 - 5 Carboxyalkyl-, 1 - 5 Ester- und/oder 1 - 3 Aminogruppe(n) substituierte geradkettige, verzweigte, gesättigte oder ungesättigte $C_1$-$C_{20}$-Alkylengruppe, wobei die gegebenenfalls enthaltenden Phenylengruppen durch 1 - 2 Carboxy-, 1 - 2 Sulfon- oder 1 - 2 Hydroxygruppen substituiert sein können,

T für eine -CO-$\alpha$, -NHCO-$\alpha$- oder -NHCS-$\alpha$-Gruppe und

$\alpha$ für die Bindungsstelle an die terminalen Stickstoffatome der letzten Generation, der Reproduktionseinheit W

stehen.

Als bevorzugte Komplexbildner-Reste K sind diejenigen genannt, bei denen in der oben angegebenen Formel IA die für $U^6$ stehende $C_1$-$C_{20}$-, bevorzugt $C_1$-$C_{12}$-, Alkylenkette die Gruppen $-CH_2-$, $-CH_2NHCO-$, $-NHCOCH_2O-$, $-NHCOCH_2OC_6H_4-$, $-N(CH_2CO_2H)-$, $-NHCOCH_2C_6H_4-$, $-NHCSNHC_6H_4-$, $-CH_2OC_6H_4-$, $-CH_2CH_2O-$, enthält und/oder durch die Gruppen $-COOH$, $-CH_2COOH$ substituiert ist.

Als Beispiele für $U^6$ sind folgende Gruppen angeführt:
$-CH_2-$, $-CH_2CH_2-$, $-CH_2CH_2CH_2-$, $-C_6H_4-$, $-C_6H_{10}-$, $-CH_2C_6H_5-$,
$-CH_2NHCOCH_2CH(CH_2CO_2H)-C_6H_4-$,
$-CH_2NHCOCH_2OCH_2-$,
$-CH_2NHCOCH_2C_6H_4-$,

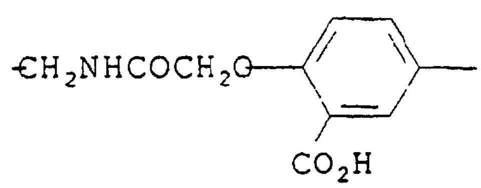
,

$-CH_2NHCSNH-C_6H_4-CH(CH_2COOH)CH_2-$,
$-CH_2OC_6H_4-N(CH_2COOH)CH_2-$,
$-CH_2NHCOCH_2O(CH_2CH_2O)_4-C_6H_4-$,
$-CH_2O-C_6H_4-$,
$-CH_2CH_2-O-CH_2CH_2-$, $-CH_2CH_2-O-CH_2CH_2-O-CH_2CH_2-$,

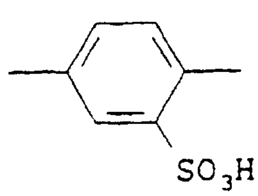

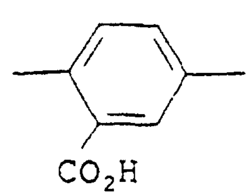

Als Beispiele für $R^4$ sind folgende Gruppen angegeben:
$-CH_3$, $-C_6H_5$, $-CH_2-COOH$,
$-CH_2-C_6H_5$, $-CH_2-O-(CH_2CH_2-O-)_6CH_3$, $-CH_2-OH$

Ist das Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21 - 29, 42, 44 und 58 - 70. Geeignete Ionen sind beispielsweise das Chrom(III)-, Eisen(II)-, Cobalt(II)-, Nickel(II)-, Kupfer (II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium(III)-, Dysprosium(III)-, Holmium(III)-, Erbium(III)-, Mangan(II)- und Eisen(III)-ion.

Ist das beschriebene Mittel zur Anwendung in der Röntgen-Diagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21 - 29, 39, 42, 44, 57 - 83 enthalten, geeignet sind; dies sind beispielsweise das Lanthan(III)-ion und die oben genannten Ionen der Lanthanidenreihe.

Die Kaskaden-Polymer-Komplexe enthalten mindestens 16 Ionen eines Elements der oben genannten Ordnungszahl.

Die restlichen aciden Wasserstoffatome, das heißt diejenigen, die nicht durch das Zentralion substituiert worden sind, können gegebenenfalls ganz oder teilweise durch Kationen von anorganischen und/oder organischen Basen, Aminosäuren oder Aminosäure-amiden ersetzt sein.

Geeignete anorganische Kationen sind beispielsweise das Lithiumion, das Kaliumion, das Calciumion, das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.

Die Verbindungen, die ein Molekulargewicht von 10.000 - 80.000 D, vorzugsweise 15.000 - 40.000 D, besitzen, weisen die eingangs geschilderten gewünschten Eigenschaften auf. Sie enthalten die für ihre Verwendung benötigte große Anzahl von Metallionen im Komplex stabil gebunden.

Sie reichern sich in Gebieten mit erhöhter Gefäßpermeabilität, wie z.B. in Tumoren, an, erlauben Aussagen über die Perfusion von Geweben, geben die Möglichkeit, das Blutvolumen in Geweben zu bestimmen, die Relaxationszeiten bzw. Densitäten des Blutes selektiv zu verkürzen, und die Permeabilität der Blutgefäße bildlich darzustellen. Solche physiologischen Informationen sind nicht durch den Einsatz von extrazellulären Kontrastmitteln, wie z.B. Gd-DTPA [Magnevist®], zu erhalten. Aus diesen Gesichtspunkten ergeben sich auch die Einsatzgebiete bei den modernen bildgebenden Verfahren Kernspintomographie und Computertomographie: spezifischere Diagnose von malignen Tumoren, frühe Therapiekontrolle bei zytostatischer, antiphlogistischer oder vaso-dilatativer Therapie, frühe Erkennung von minderperfundierten Gebieten (z.B. im Myokard), Angiographie bei Gefäßerkrankungen, und Erkennung und Diagnose von (sterilen oder infektiösen) Entzündungen.

Die beschriebenen Kaskaden-Polymer-Komplexe eignen sich auch hervorragend für die (interstitielle und i. v.) Lymphographie.

Als weitere Vorteile gegenüber extrazellulären Kontrastmitteln, wie z.B. Gd-DTPA [Magnevist®], muß die höhere Effektivität als Kontrastmittel für die Kernspintomographie (höhere Relaxivität) hervorgehoben werden, was zu einer deutlichen Reduktion der diagnostisch notwendigen Dosis führt. Gleichzeitig können die beschriebenen Kontrastmittel als Lösungen isoosmolar zum Blut formuliert werden und verringern dadurch die osmotische Belastung des Körpers, was sich in einer verringerten Toxizität der Substanz (höhere toxische Schwelle) niederschlägt. Geringere Dosen und höhere toxische Schwelle führen zu einer signifikanten Erhöhung der Sicherheit von Kontrastmittelanwendungen bei modernen bildgebenden Verfahren.

Im Vergleich zu den makromolekularen Kontrastmitteln auf der Basis von Kohlen-hydraten, z.B. Dextran (Europäische Patentanmeldung, Publikations-Nr. 0 326 226), die- wie erwähnt - in der Regel nur ca. 5 % des signalverstärkenden paramagnetischen Kations tragen, weisen die Polymer-Komplexe einen Gehalt von in der Regel ca 20 % des paramagnetischen Kations auf Somit bewirken die beschriebenen Makromoleküle pro Molekül eine sehr viel höhere Signalverstärkung, was gleichzeitig dazu führt, daß die zur Kernspintomographie notwendige Dosis gegenüber der makro-molekularer Kontrastmittel auf Kohlenhydratbasis erheblich kleiner ist.

Diese Polymerkomplexe sind groß genug um den vasalen Raum nur langsam verlassen zu können, aber gleichzeitig klein genug, die Kapillaren der Niere, welche 300 - 800 Å groß sind, noch passieren zu können.

Im Vergleich zu den anderen erwähnten Polymer-Verbindungen des Stands der Technik zeichnen sich die beschriebenen Kaskaden-Polymer-Komplexe durch verbessertes Ausscheidungsverhalten, höhere Wirksamkeit, größere Stabilität und/oder bessere Verträglichkeit aus.

Die Herstellung der makrocyclische Kaskaden-Polymer-Komplexe erfolgt dadurch, daß man Verbindungen der allgemeinen Formel I'

$$A\text{-}\{X\text{-}[Y\text{-}(Z\text{-}\langle W\text{-}\beta_w \rangle_z)_y]_x\}_a \qquad (I'),$$

worin

| | |
|---|---|
| A | für einen stickstoffhaltigen Kaskadenkern der Basismultiplizität a, X und Y unabhängig voneinander für eine direkte Bindung oder eine Kaskadenreproduktionseinheit der Reproduktionsmultiplizität x bzw. y, |
| Z und W | unabhängig voneinander für eine Kaskadenreproduktionseinheit der Reproduktionsmultiplizität z bzw. w, |
| a | für die Ziffern 2 bis 12, |
| x, y, z und w | unabhängig voneinander für die Ziffern 1 bis 4 und |
| β | für die Bindungsstelle der terminalen NH-Gruppen der letzten Generation, der Reproduktionseinheit W stehen mit der Maßgabe, daß mindestens zwei Reproduktionseinheiten unter schiedlich sind und daß für das Produkt der Multiplizitäten gilt $16 \leq a \cdot x \cdot y \cdot z \cdot w \leq 64$, |

mit einem Komplex oder Komplexbildner K' der allgemeinen Formel I'A

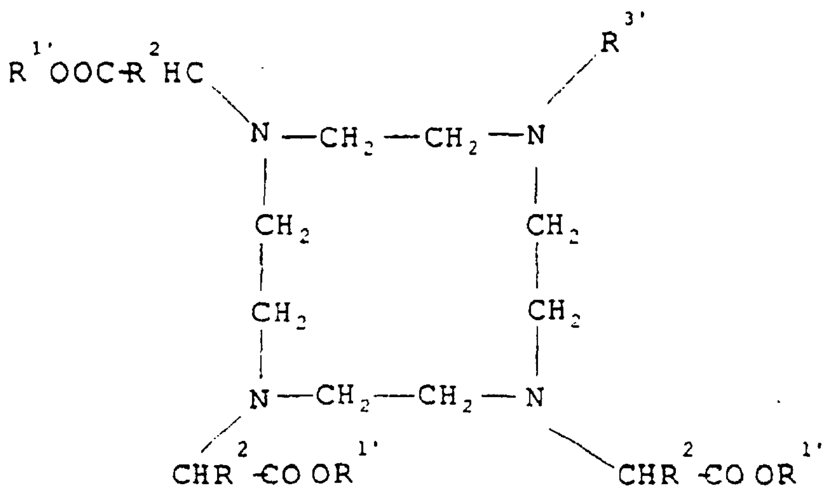

(I

wobei

R1' unabhängig voneinander für ein Wasserstoffatom, ein Metallionen-äquivalent der Ordnungszahlen 20 - 29, 39, 42 - 44 oder 57 - 83 oder eine Säureschutzgruppe,

$R^2$ für ein Wasserstoffatom, einen Methyl- oder einen Ethylrest, der gegebenenfalls mit 1 - 2 Hydroxy- oder 1 Carboxygruppe(n) substituiert ist,

$R^{3'}$ für eine

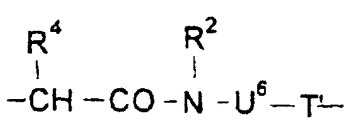

Gruppe

$R^4$ für eine geradkettige, verzweigte, gesättigte oder ungesättigte $C_1$-$C_{30}$-Alkylkette, die gegebenenfalls durch 1 - 10 Sauerstoffatome, 1 Phenylen-, 1 Phenylenoxygruppen unterbrochen und/oder gegebenenfalls durch 1 - 5 Hydroxy-, 1 - 3 Carboxy-, 1 Phenylgruppe(n) substituiert ist,

$U^6$ für eine gegebenenfalls 1 - 5 Imino-, 1 - 3 Phenylen-, 1 - 3 Phenylenoxy-, 1 - 3 Phenylenimino-, 1 - 5 Amid-, 1 - 2 Hydrazid-, 1 - 5 Carbonyl-, 1 - 5 Ethylen oxy-, 1 Harnstoff-, 1-Thioharnstoff-, 1 - 2 Carboxyalkylimino-, 1 - 2 Estergruppen, 1 - 10 Sauerstoff-, 1 - 5 Schwefel- und/oder 1 - 5 Stickstoff-Atom(e) enthaltende und/oder gegebenenfalls durch 1 - 5 Hydroxy-, 1 - 2 Mercapto-, 1 - 5 Oxo-, 1 - 5 Thioxo-, 1 - 3 Carboxy-, 1 - 5 Carboxyalkyl-, 1 - 5 Ester- und/oder 1 - 3 Aminogruppe(n) substituierte geradkettige, verzweigte, gesättigte oder ungesättigte $C_1$-$C_{20}$-Alkylengruppe, wobei die gegebenenfalls enthaltenden Phenylengruppen durch 1 - 2 Carboxy-, 1 - 2

Sulfon- oder 1 - 2 Hydroxygruppen substituiert sein können,

T' für eine -C*O-, -COOH-, -N=C=O- oder -N=C=S-Gruppe und

C*O für eine aktivierte Carboxylgruppe

stehen
mit der Maßgabe, daß - sofern K' für einen Komplex steht - mindestens zwei (bei zweiwertigen Metallen) bzw. drei (bei dreiwertigen Metallen) der Substituenten $R^1$ für ein Metallionenäquivalent der oben genannten Elemente stehen und daß gewünschtenfalls weitere Carboxylgruppen in Form ihrer Salze mit anorganischen und/oder organischen Basen, Aminosäuren oder Aminosäureamiden vorliegen,
umsetzt, gegebenenfalls vorhandene Schutzgruppen abspaltet, die so erhaltenen Kaskaden-Polymere - sofern K' für einen Komplexbildner steht - in an sich bekannter Weise mit mindestens einem Metalloxid oder Metallsalz eines Elements der Ordnungszahlen 20 - 29, 39, 42, 44 oder 57 - 83 umsetzt und gegebenenfalls anschließend in den so erhaltenen Kaskaden-Polymer-Komplexen noch vorhandene acide Wasserstoffatome ganz oder teilweise durch Kationen von anorganischen und/oder organischen Basen, Aminosäuren oder Aminosäureamiden substituiert und gegebenenfalls noch vorhandene freie terminale Aminogruppen gewünschtenfalls - vor oder nach der Metallkomplexierung - acyliert.

Offenbart wird die Umsetzung mit Komplexbildnern der allgemeinen Formel I'A, in der Bedeutung von $R^{1'}$ = t-butyl.

Die Herstellung der Komplexe und Komplexbildner der allgemeinen Formel I'A erfolgt nach bzw. analog den im experimentellen Teil beschriebenen Vorschriften bzw. nach literaturbekannten Methoden (s. z.B. Europäische Patentanmeldungen Nr. 0 512 661, 0 430 863, 0 255 471 und 0 565 930.

So kann die Herstellung von Verbindungen der allgemeinen Formel I'A z.B. dadurch erfolgen, daß als Vorstufe der funktionellen Gruppe T' eine Gruppe T" dient, entweder in der Bedeutung einer geschützten Säurefunktion, die unabhängig von den Säureschutzgruppen $R^{1'}$ nach den oben aufgeführten Verfahren in die freie Säurefunktion überführt werden kann, oder in der Bedeutung einer geschützten Aminfunktion, die nach literaturbekannten Verfahren deblockiert [Th.W. Greene, P.G.M. Wuts, Protective Groups in Organic Synthesis, 2nd edition, John Wiley & Sons (1991), S. 309-385] und anschließend in die Isocyanate bzw. Isothiocyanate überführt werden kann [Methoden der Org. Chemie (Houben-Weyl), E 4, S. 742-749, 837-843, Georg Thieme Verlag, Stuttgart, New York (1983)]. Solche Verbindungen sind nach bzw. analog den im experimentellen Teil beschriebenen Vorschriften durch Monoalkylierung von Cyclen mit geeigneten $\alpha$-Halogencarbonsäureamiden [in aprotischen Lösungsmitteln, wie z.B. Chloroform] herstellbar.

Hinsichtlich weiterer Einzelheiten über die Kopplungsreaktionen, Ausgangssubstanzen, Einführung der gewünschten Metallionen, Herstellung und Verabreichung der pharmazeutischen Mittel etc. sei auf die WO 96/01655, insbesondere die Seiten 22 bis 33 verwiesen.

Die vorliegende Erfindung betrifft neue makrocyclische Metallkomplexcarbonsäuren der Formel II

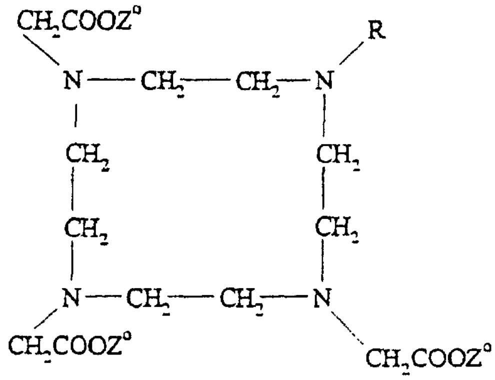

(II),

wobei

Z° für ein Metallionenäquivalent der Ordnungszahlen 58-71 und

R für eine $CHX^1$-CO-NH-$CHY^1$-$(CH_2)_f$-COOH-Gruppe steht, worin $X^1$ und $Y^1$ unabhängig voneinander einen geradkettigen oder verzweigten $C_1$-$C_7$-Alkylrest, eine Phenyl- oder Benzylgruppe und $Y^1$ zusätzlich ein Wasserstoffatom und f die Ziffern 0 bis 9 bedeuten,

die als Zwischenprodukte für die Synthese von Kaskaden-Polymer-Komplexe der allgemeinen Formel I der PCT/EP 96/02671 und der PCT/EP 95/02577 eingesetzt werden können.
Keine der in EP-A-430863, DE-A-19549286, DE-A-19525924 und DE-A-4344460 beschriebenen Synthesen von Kaskaden-Polymer-Komplexen verwendet die erfindungsgemäßen Metallkomplexcarbonsäuren der Formel II.
Für die Reste $X^1$ bzw. $Y^1$ seien beispielhaft genannt: Methyl, Ethyl, Propyl, Butyl bzw. Wasserstoff, Methyl, Isopropyl, Phenyl und Benzyl. Bevorzugt sind Methyl bzw. Wasserstoff
Der Index f steht bevorzugt für die Ziffern 0,1 oder 2.

Von den oben genannten Lanthaniden sind Gadolinium, Dysprosium und Ytterbium bevorzugt.

Die Umsetzung der neuen makrocyclischen Metallkomplexcarbonsäuren der Formel II zu den gewünschten Kaskaden-Polymer-Komplexen erfolgt analog literaturbekannter Methoden, z.B. B. Belleau, G. Malek, J. Amer.Chem. Soc. 90, 1651 (1968). Die so erhaltenen Produkte weisen eine geringere Nebenproduktverteilung auf als die ohne Verwendung der erfindungsgemäßen Metallkomplexcarbonsäuren der Formel II synthetisierten Kaskaden-Polymer-Komplexe.

Die Synthese der erfindungsgemäßen Verbindungen der allgemeinen Formel II erfolgt dadurch, daß man Verbindungen der allgemeinen Formel III

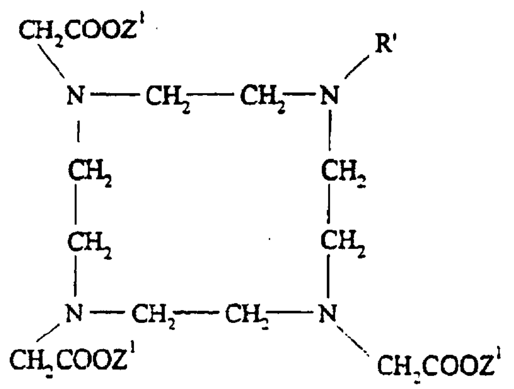

(III),

worin

R' die Bedeutung von R hat, wobei die darin enthaltene Carboxylgruppe gegebenenfalls in geschützter Form vorliegt und

$Z^1$ für ein Wasserstoffatom oder eine Carboxylschutzgruppe steht, nach Abspaltung der gegebenenfalls vorhandenen Carboxylschutzgruppen, in an sich bekannter Weise mit einem Metalloxid oder Metallsalz eines Elements der Ordnungszahlen 58-71 umsetzt.

Die Einführung der gewunschten Metallionen erfolgt in der Weise, wie sie z.B. in den Patentschriften EP 71564, EP 130934 und DE-3401052 offenbart worden ist, in dem man das Metalloxid oder ein Metallsalz (beispielsweise das Nitrat, Acetat, Carbonat, Chlorid oder Sulfat) des Elements der Ordnungszahlen 58-71 in Wasser und/oder einem niederen Alkohol (wie Methanol, Ethanol oder Isopropanol) löst oder suspendiert und mit einer Lösung oder Suspension der äquivalenten Menge des Komplexbildners der allgemeinen Formel III umsetzt.

Falls $Z^1$ für eine Säureschutzgruppe steht, kommen z.B. geradkettige oder verzweigte $C_1$-$C_6$-Alkyl-, Aryl- und Aralkylgruppen, beispielsweise die Methyl-, Ethyl-, Propyl-, Butyl-, Phenyl-, Benzyl-, Diphenylmethyl-, Triphenylmethyl-, bis(p-Nitrophenyl)-methylgruppe sowie Trialkylsilylgruppen in Frage. Bevorzugt ist die t-Butylgruppe.

Die Abspaltung der Schutzgruppen erfolgt nach den dem Fachmann bekannten Verfahren, beispielsweise durch Hydrolyse, Hydrogenolyse, alkalische Verseifung der Ester mit Alkali in wässrig-alkoholischer Lösung bei Temperaturen von 0° bis 50° C, saure Verseifung mit Mineralsäuren oder im Fall von z.B tert.-Butylestern mit Hilfe von Trifluoressigsäure.[Protective Groups in Organic Synthesis, 2nd Edition, T.W. Greene and P.G.M. Wuts, John Wiley and Sons, Inc. New York, 1991].

Verbindungen der allgemeinen Formel III kann man erhalten durch Umsetzen von $\alpha$-Halogencarbonsäureestern oder -säuren der allgemeinen Formel IV

$$Hal\text{-}CH_2\text{-}CO_2Z^1 \qquad (IV),$$

worin

$Z^1$ die obengenannte Bedeutung hat und Hal für Chlor- Brom oder Iod steht,

mit Verbindungen der allgemeinen Formel V

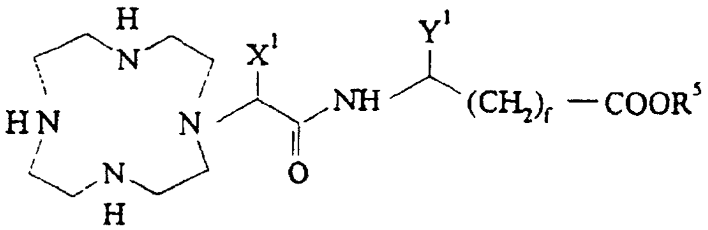

(V),

worin

$R^5$ für ein Wasserstoffatom, oder eine Säureschutzgruppe steht und
$X^1$, $Y^1$ und f die oben genannte Bedeutung haben.

Stehen $Z^1$ und $R^5$ jeweils für eine Säureschutzgruppe so können diese unterschiedliche Bedeutung haben, so daß $Z^1$ (z.B. Benzyl) gegebenenfalls selektiv (z.B. durch Hydrolyse) in Gegenwart von $R^5$-Schutzgruppen (z.B. t-Butyl) abgespalten werden kann.

Steht $Z^1$ für eine Säureschutzgruppe, so erfolgt die Umsetzung bevorzugt in Lösungsmitteln wie Methylenchlorid, Dimethylformamid, Acetonitril, Tetrahydrofuran, Dioxan, Chloroform, niederen Alkoholen wie Methanol, Ethanol und Isopropanol, sowie Mischungen aus den oben genannten Lösungsmitteln mit Wasser.

Bei Verwendung einer Halogencarbonsäure als Edukt wird bevorzugt in Wasser gearbeitet.

Als Säurefänger dienen organische Basen wie Pyridin, Triethylamin oder Diisopropylethylamin oder anorganische Basen wie Natriumhydroxid, Lithiumhydroxid, Kaliumhydroxid, Calciumhydroxid oder Natriumcarbonat, Kaliumcarbonat, Natriumhydrogencarbonat oder Lithiumcarbonat. Die Alkylierung wird bei Temperaturen zwischen 0-100° C durchgeführt, bevorzugt jedoch bei 20-80° C.

Verbindungen der allgemeinen Formel V werden erhalten durch Umsetzung von Cyclen (Formel VI)

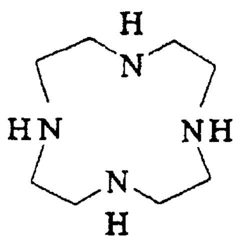

(VI),

mit Verbindungen der allgemeinen Formel VII

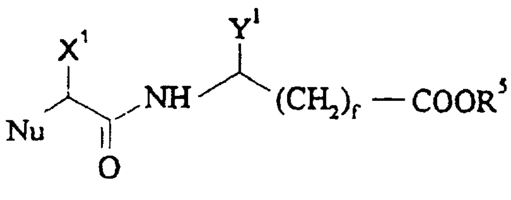

(VII),

worin

$X^1$, $Y^1$, $R^5$ und fdie oben genannte Bedeutung haben und Nu für ein Nucleofug steht. Als Nucleofuge seien Chlorid, Bromid, Jodid, Mesylat, Tosylat oder Triflat genannt.

Die Umsetzung erfolgt in Lösungsmitteln wie Chloroform, Methylenchlorid, Tetrahydrofuran, Dioxan, Dimethylformamid, Dimethylsulfoxid oder auch in Wasser bei Temperaturen von 0° C bis 100° C, bevorzugt jedoch bei 20°

- 60° C. Es kann, falls erwünscht, eine organische oder anorganische Base zugesetzt werden. Beispielhaft genannt seien Triethylamin, Pyridin, Natriumcarbonat, Natriumhydroxid oder Kaliumhydroxid.

Verbindungen der allgemeinen Formel VII erhält man durch Umsetzen von Verbindungen der allgemeinen Formel VIII

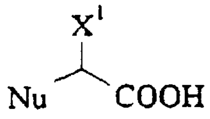 (VIII),

worin

Nu und $X^1$ die oben genannte Bedeutung haben, mit Verbindungen der allgemeinen Formel IX

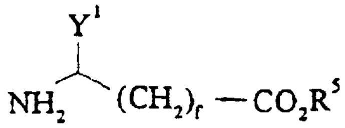 (IX),

worin

$Y^1$, f und $R^5$ die oben angegebene Bedeutung haben.

Die Umsetzung erfolgt nach den dem Fachmann bekannten Methoden aus der Peptidchemie. So kann beispielsweise aus der Säure der allgemeinen Formel VIII ein Derivat wie z.B. ein Säurechlorid, Säurebromid oder Aktivester (wie z.B. NHS-Ester) hergestellt weden, das mit einer Aminosäure (gegebenenfalls terminalgeschützt) kondensiert wird.

Verbindungen der allgemeinen Formel VIII, sowie deren Säurechloride und Säurebromide sind käuflich erhältlich. Verbindungen der allgemeinen Formel IX sind ebenfalls als freie Aminosäuren oder in geschützter Form käuflich erhältlich.

Alternativ können Verbindungen der allgemeinen Formel III durch Umsetzung von Verbindungen der allgmeinen Formel X

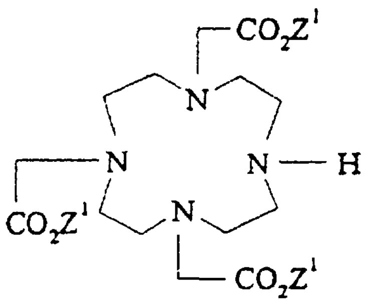 (X),

worin $Z^1$ die oben genannte Bedeutung besitzt, mit Verbindungen der allgemeinen Formel VII, nach Abspaltung der gegebenenfalls vorhandenen Säureschutzgruppen, erhalten werden.

Die Umsetzung erfolgt in Lösungsmitteln wie beispielsweise Acetonitril, Dimethylformamid, Tetrahydrofuran, Dioxan oder niederen Alkoholen wie Methanol, Ethanol oder i-Propanol sowie Mischungen dieser mit Wasser; es kann aber auch in reinem Wasser gearbeitet werden. Man arbeitet im allgemeinen bei Temperaturen von 20° C - 100° C.

Als Säurefänger werden organische oder anorganische Basen verwendet. Beispielhaft genannt seien Triethylamin, Pyridin, 4-Dimethylaminopyridin, Natriumhydroxid, Kaliumhydroxid, Kaliumcarbonat, Natriumcarbonat. Es können auch Metallhydride wie Natriumhydrid, Calciumhydrid eingesetzt werden, jedoch nur bei aprotischen Lösungsmitteln.

Der Zusatz katalytischer Mengen eines Iodids hat sich als vorteilhaft erwiesen. Beispielhaft genannt seien

Natriumiodid, Kaliumiodid, Lithiumiodid oder Tetrabutylammoniumiodid.

Die Reinigung der erfindungsgemäßen Metallkomplexe der allgemeinen Formel II erfolgt beispielsweise durch Chromatographie an Kieselgel oder RP-18.

Die meisten der erfindungsgemäßen Metallkomplexe der allgemeinen Formel II können aus Alkoholen wie Methanol, Ethanol oder Isopropanol kristallisiert werden oder auch aus deren Mischungen mit Wasser.

Es hat sich auch als günstig erwiesen die erfindungsgemäßen Metallkomplexe in Alkoholen oder Mischungen von Alkoholen mit Wasser zu lösen und durch Zutropfen von Aceton auszufällen.

Das Trocknen der erfindungsgemäßen Metallkomplexcarbonsäuren geschieht vorteilhafter Weise im Vakuum bei Temperaturen von 20° - 200° C, bevorzugt 50° - 130° C, innerhalb von ca. 6 Stunden bis zu 3 Tagen.

Die so erhaltenen Metallkomplexcarbonsäuren der allgemeinen Formel II werden unter Feuchtigkeitsausschluß gelagert und können direkt in eine Kupplungsreaktion eingesetzt werden.

Insgesamt ist es gelungen mit den erfindungsgemäßen Metallkomplexcarbonsäuren der allgemeinen Formel II wichtige Zwischenprodukte zur Verfügung zu stellen, die es erlauben, Kaskadenpolymerkomplexe mit geringerem Nebenproduktanteil zu synthetisieren.

Die nachfolgenden Beispiele 1 bis 3 dienen zur Erläuterung der Synthese von Polymer-Komplexen mittels Kupplung der makrocyclischen Liganden, wie in der PCT/EP 96/02671 beschrieben.

**Beispiel 1**

a) Bis[2-(benzyloxycarbonylamino)-ethyl]-amin

51,5 g (500 mmol) Diethylentriamin und 139 ml (1 mol) Triethylamin werden in Dichlormethan gelöst und bei -20°C mit 161 g Benzylcyanformiat (Fluka) in Dichlormethan versetzt und anschließend über Nacht bei Raumtemperatur gerührt. Nach Beendigung der Reaktion wird im Abzug eingedampft, der Rückstand in Diethylether aufgenommen, die organische Phase mit Natriumcarbonatlösung gewaschen und mit Natriumsulfat getrocknet. Das Filtrat wird mit Hexan versetzt, der Niederschlag abfiltriert und getrocknet.
Ausbeute: 163,4 g (88 % d. Th.)

| Elementaranalyse: | | | |
|---|---|---|---|
| ber | C 64,67 | H 6,78 | N 11,31 |
| gef. | C 64,58 | H 6,83 | N 11,28 |

b) N,N,N',N',N",N"-Hexakis[2-(benzyloxycarbonylamino)-ethyl]-trimesinsäuretriamid

13,27 g (50 mmol) Trimesinsäure-trichlorid (Aldrich) und 34,7 ml (250 mmol) Triethylamin werden in Dimethylformamid (DMF) gelöst und bei 0°C mit 65,0 g (175 mmol) des in Beispiel 1a) beschriebenen Amins versetzt und anschließend über Nacht bei Raumtemperatur gerührt. Die Lösung wird im Vakuum eingedampft und der Rückstand mit Ethylacetat an Kieselgel chromatographiert.
Ausbeute: 39,4 g (62 % d. Th.)

| Elementaranalyse: | | | |
|---|---|---|---|
| ber. | C 65,24 | H 5,95 | N 9,92 |
| gef. | C 65,54 | H 5,95 | N 9,87 |

c) $N^{\alpha}$, $N^{\varepsilon}$-Bis(N,N'-dibenzyloxycarbonyl-lysy)-lysin, geschütztes "Tri-Lysin"

3,6 g (20 mmol) Lysin-Hydrochlorid und 6,95 ml (50 mmol) Triethylamin werden in DMF gelöst, mit 26,8 g (50 mmol) $N^{\alpha}$, $N^{\varepsilon}$-Dibenzyloxycarbonyl-Lysin-p-nitrophenylester (Bachem) versetzt und 2 Tage bei Raumtemperatur gerührt. Nach Beendigung der Reaktion wird im Vakuum eingedampft, der Rückstand in Ethylacetat aufgenommen und mit verdünnter Salzsäure ausgeschüttelt. Die organische Phase wird mit Natriumsulfat getrocknet, das Lösungsmittel eingedampft und der Rückstand mit Ethylacetat/Ethanol in einem Stufengradienten chromatographiert.
Ausbeute: 10,7 g (57 % d. Th.)

| Elementaranalyse: | | | |
|---|---|---|---|
| ber. | C 63,95 | H 6,65 | N 8,95 |

(fortgesetzt)

| Elementaranalyse: | | | |
|---|---|---|---|
| gef. | C 63,63 | H 6,69 | N 8,93 |

d) Vollgeschütztes Benzyloxycarbonyl-24mer-Polyamin auf der Basis des N,N,N',N',N",N"-Hexakis[2-(trilysyl-amino)-ethyl]-trimesinsäuretriamids

1,27 g (1 mmol) des im Beispiel 1b) beschriebenen Hexa-Benzyloxycarbonylamins werden in Eisessig gelöst und unter Rühren mit 33 %igem Bromwasserstoff in Eisessig versetzt. Nach 60 Minuten wird mit Diethylether die begonnene Fällung vervollständigt, das entstandene Hexaaminhydrobromid mit Ether gewaschen, im Vakuum getrocknet und ohne weitere Reinigung in die weiter unten beschriebene Reaktion eingesetzt.
Ausbeute: 0,95 g (quantitativ)

7,0 g (7,5 mmol) des in Beispiel 1c) beschriebenen geschützten "Tri-Lysins", 1,2 g (7,5 mmol) 1-Hydroxybenzotriazol und 2,4 g (7,5 mmol) 2-(1H-Benzotriazol-1-yl)-1,1,3,3-tetramethyluronium tetrafluorborat (TBTU; Peboc Limited, UK) werden in DMF gelöst und 15 Minuten gerührt. Diese Lösung wird anschließend mit 5,16 ml (30 mmol) N-Ethyldiisopropylamin und mit 0,95 g (1 mmol) des oben beschriebenen Hexaaminhydrobromids versetzt und über Nacht bei Raumtemperatur gerührt. Nach Beendigung der Reaktion wird im Vakuum eingedampft und der Rückstand mit Ethylacetat/Ethanol (2:1) an Kieselgel chromatographiert.
Ausbeute: 4,55 g (76 % d. Th.)

| Elementaranalyse: | | | |
|---|---|---|---|
| ber. | C 64,35 | H 6,71 | N 10,52 |
| gef. | C 64,08 | H 6,57 | N 10,29 |

e) N-(2-Brompropionyl)glycin-benzylester

Zu 100 g (296,4 mmol) Glycinbenzylester-p-Toluolsulfonsäuresalz und 33,0 g (326,1 mmol) Triethylamin in 400 ml Methylenchlorid tropft man bei 0 °C 55,9 g (326,1 mmol) 2-Brompropionsäurechlorid zu. Man läßt die Temperatur nicht über 5 °C kommen. Nach beendeter Zugabe wird eine Stunde bei 0 °C gerührt, anschließend 2 Stunden bei Raumtemperatur. Man setzt 500 ml Eiswasser zu und stellt die Wasserphase mit 10 % aqu. Salzsäure auf pH 2. Die organische Phase wird abgetrennt, je einmal mit 300 ml 5 % aqu. Sodalösung und 400 ml Wasser gewaschen. Man trecknet die organische Phase über Magnesiumsulfat und dampft im Vakuum zur Trockne ein. Der Rückstand wird aus Diisopropylether umkristallisiert.
Ausbeute: 68,51 g (75 % d. Th.) eines farblosen kristallinen Pulvers
Schmelzpunkt: 69-70°C

| Elementaranalyse: | | | | |
|---|---|---|---|---|
| ber. | C 48,02 | H 4,70 | N 4,67 | Br 26,62 |
| gef. | C 47,91 | H 4,82 | N 4,51 | Br 26,47 |

f) 1-[4-(Benzyloxycarbonyl)-1-methyl-2-oxo-3-azabutyl]-1,4,7,10-tetraazacyclododecan

Zu 55,8 g (324,4 mmol) 1,4,7,10-Tetraazacyclododecan, gelöst in 600 ml Chloroform, gibt man 50 g (162,2 mmol) der Titelverbindung aus Beispiel 1e) und rührt über Nacht bei Raumtemperatur. Man gibt 500 ml Wasser zu, trennt die organische Phase ab und wäscht sie noch jeweils 2 mal mit 400 ml Wasser. Man trocknet die organische Phase über Magnesiumsulfat und dampft im Vakuum zur Trockne ein. Der Rückstand wird an Kieselgel chromatographiert (Laufmittel: Chloroform/Methanol/aqu. 25 % Ammoniak = 10/5/1). Ausbeute. 40,0 g [63 % d. Th.bezogen auf eingesetztes 1e)] eines leicht gelblichen zähen Öls.

| Elementaranalyse: | | | |
|---|---|---|---|
| ber. | C 61,36 | H 8,50 | N 17,89 |
| gef. | C 61,54 | H 8,68 | N 17,68 |

g) 10-[4-(Benzyloxycarbonyl)-1-methyl-2-oxo-3-azabutyl]-1,4,7-tris(tert-butoxycarbonylmethyl)-1,4,7,10-tetraazacyclododecan (Natriumbromid-Komplex)

Zu 20 g (51,08 mmol) der Titelverbindung aus Beispiel 1f) und 17,91 (169 mmol) Natriumcarbonat in 300 ml Acetonitril gibt man 33 g (169 mmol) Bromessigsäure-tert.-butylester zu und rührt 24 Stunden bei 60 °C. Man kühlt auf 0 °C ab, filtriert von den Salzen ab und dampft das Filtrat zur Trockne ein. Der Rückstand wird an Kieselgel chromatographiert (Laufmittel: Essigsäureethylester/Ethanol: 15/1). Die das Produkt enthaltenden Fraktionen werden eingedampft und der Rückstand aus Diisopropylether umkristallisiert.
Ausbeute: 34,62 g (81 % d. Th.) eines farblosen kristallinen Pulvers
Schmelzpunkt: 116 - 117 °C

| Elementaranalyse: | | | | | |
|---|---|---|---|---|---|
| ber | C 54,54 | H 7,59 | N 8,37 | Na 2,74 | Br 9,56 |
| gef. | C 54,70 | H 7,65 | N 8,24 | Na 2,60 | Br 9,37 |

h) 10-(4-Carboxy-1-methyl-2-oxo-3-azabutyl)-1,4,7-tris(tert.-butoxycarbonylmethyl)-1,4,7,10-tetraazacyclododecan (Natriumbromid-Komplex)

30 g (35,85 mmol) der Titelverbindung aus Beispiel 1g werden in 500 ml Isopropanol gelöst und 3 g Palladiumkatalysator (10 % Pd/C) hinzugegeben. Man hydriert über Nacht bei Raumtemperatur. Es wird vom Katalysator abfiltriert, das Filtrat im Vakuum zur Trockne eingedampft und aus Aceton umkristallisiert.
Ausbeute: 22,75 g (85 % d. Th.) eines farblosen kristallinen Pulvers
Schmelzpunkt: 225 °C (Zers.)

| Elementaranalyse: | | | | | |
|---|---|---|---|---|---|
| ber. | C 49,86 | H 7,69 | N 9,38 | Na 3,07 | Br 10,71 |
| gef. | C 49,75 | H 7,81 | N 9,25 | Na 2,94 | Br 10,58 |

i) 24-mer N-(5-DO3 A-yl-4-oxo-3-azahexanoyl)-Kaskadenpolyamid auf der Basis des N,N,N',N',N",N"-Hexakis[2-(trilysylamino)-ethyl]-trimesinsäuretriamids *)

6,0 g (1 mmol) des in Beispiel 1d) beschriebenen Poly-Benzyloxycarbonylamins werden in Eisessig gelöst und unter Rühren mit 33 %igem Bromwasserstoff in Eisessig versetzt. Nach 3 Stunden wird mit Diethylether die begonnene Fällung vervollständigt, das entstandene 24-Amin-hydrobromid mit Ether gewaschen und im Vakuum getrocknet.

35,84 g (48 mmol) der im vorstehenden Beispiel 1h) beschriebenen Säure werden in DMF gelöst, mit 7,35 g (48 mmol) 1-Hydroxybenzotriazol, mit 15,41 g (48 mmol) TBTU (Peboc Limited, UK) und mit 49,3 ml (288 mmol) N-Ethyldiisopropylamin versetzt und 20 Minuten bei Raumtemperatur gerührt. Diese Lösung wird anschließend mit dem oben beschriebenen (1 mmol) 24-Aminhydrobromid versetzt und 4 Tage bei Raumtemperatur gerührt. Die Lösung wird im Vakuum eingeengt, das verbleibende Öl im Eisbad gekühlt und mit Trifluoressigsäure versetzt, über Nacht bei Raumtemperatur gerührt und anschließend mit Diethylether gefällt. Der Niederschlag wird im Vakuum getrocknet, in Wasser aufgenommen, auf pH 7 eingestellt, über eine YM3 Amicon® -Ultrafiltrationsmembran von niedermolekularen Anteilen gereinigt und das Retentat schließlich membranfiltriert und gefriergetrocknet.
Ausbeute: 13,5 g (83 % d.Th.)
$H_2O$-Gehalt (Karl-Fischer): 6,2 %

| Elementaranalyse (bezogen auf wasserfreie Substanz): | | | | |
|---|---|---|---|---|
| ber. | C 45,82 | H 6,09 | N 15,07 | Na 10,79 |
| gef. | C 45,56 | H 6,15 | N 14,80 | Na 10,52 |

k) 24-mer-Gd-Komplex des N-(5-DO3A-yl-4-oxo-3-azahexanoyl)-Kaskadenpolyamids auf der Basis des N,N,N',N',N",N"-Hexakis[2-(trilysylamino)-ethyl]-trimesinsäuretriamids

8,13 g (0,5 mmol) der im vorstehenden Beispiel 1i) beschriebenen Komplexbildnersäure werden in Wasser

*) DO3A= 1,4,7-Tris(carboxymethyl)-1,4,7,10-tetraazacyclododecan

mit verd. Salzsäure auf pH 3 gestellt, mit 2,17 g (6 mmol) $Gd_2O_3$ versetzt, 30 Minuten bei 80 °C gerührt, nach dem Abkühlen auf pH 7 eingestellt und über eine YM3 AMICON®-Ultrafiltrationsmembran entsalzt. Das Retentat wird schließlich membranfiltriert und gefriergetrocknet
Ausbeute: 8,89 g (92,1 % d. Th.)
$H_2O$-Gehalt (Karl-Fischer): 9,6 %
Gd-Bestimmung (AAS): 19,6 %

| Elementaranalyse (bezogen auf wasserfreie Substanz): | | | | |
|---|---|---|---|---|
| ber. | C 40,26 | H 5,35 | N 13,24 | Gd 21,62 |
| gef. | C 39,98 | H 5,51 | N 13,42 | Gd 21,37 |

**Beispiel 2**

a) 2-Brompropionyl-β-alanin-benzylester

Zu 100 g (285 mmol) β-Alaninbenzylester-p-Toluolsulfonsäuresalz und 31,67 g (313 mmol) Triethylamin in 400 ml Methylenchlorid tropft man bei 0 °C 53,65 g (313 mmol) 2-Brompropionsäurechlorid zu. Man läßt die Temperatur nicht über 5 °C kommen. Nach beendeter Zugabe wird 1 Stunde bei 0 °C gerührt, anschließend 2 Stunden bei Raumtemperatur. Man setzt 500 ml Eiswasser zu und stellt die Wasserphase mit 10 % aqu. Salzsäure auf pH 2. Die organische Phase wird abgetrennt, je einmal mit 300 ml 5 % aqu. Salzsäure, 300 ml 5 % aqu. Sodalösung und 400 ml Wasser gewaschen. Man trocknet die organische Phase über Magnesiumsulfat und dampft im Vakuum zur Trockne ein. Der Rückstand wird aus Diisopropylether umkristallisiert.
Ausbeute: 71,36 g (78 % d. Th.) eines farblosen kristallinen Pulvers

| Elementaranalyse: | | | | |
|---|---|---|---|---|
| ber. | C 48,46 | H 7,51 | N 4,35 | Br 24,80 |
| gef. | C 48,29 | H 7,65 | N 4,25 | Br 24,61 |

b) 1-[5-(Benzyloxycarbonyl)-1-methyl-2-oxo-3-azapentyl]-1,4,7,10-tetraazacyclododecan

Zu 53,32 g (310 mmol) 1,4,7,10 Tetraazacyclododecan gelöst, in 600 ml Chloroform, gibt man 50 g (155,2 mmol) der Titelverbindung aus Beispiel 2a) und rührt über Nacht bei Raumtemperatur. Man gibt 500 ml Wasser zu, trennt die organische Phase ab und wäscht sie noch jeweils 2 mal mit 400 ml Wasser. Man trocknet die organische Phase über Magnesiumsulfat und dampft im Vakuum zur Trockne ein. Der Rückstand wird an Kieselgel chromatographiert (Laufmittel: Chloroform/Methanol/aqu. 25 % Ammoniak: 10/5/1). Ausbeute: 38,39 g [61 % d. Th.bezogen auf eingesetztes 2a)] eines leicht gelblichen zähen Öls.

| Elementaranalyse: | | | |
|---|---|---|---|
| ber. | C 62,20 | H 8,70 | N 17,27 |
| gef. | C 62,05 | H 8,81 | N 17,15 |

c) 10-[5-(Benzyloxycarbonyl)-1-methyl-2-oxo-3-azapentyl]-1,4,7-tris(tert.-butoxycarbonylmethyl)-1,4,7,10-tetraazacyclododecan (Natriumbromid-Komplex)

Zu 20 g (49,32 mmol) der Titelverbindung aus Beispiel 2b) und 17,28 g (163 mmol) Natriumcarbonat in 300 ml Acetonitril gibt man 31,8 g (163 mmol) Bromessigsäure-tert.butylester zu und rührt 24 Stunden bei 60 °C. Man kühlt auf 0 °C ab, filtriert von den Salzen ab und dampft das Filtrat zur Trockne ein. Der Rückstand wird an Kieselgel chromatographiert (Laufmittel: Essigsäureethylester/Ethanol = 10/1). Die das Produkt enthaltenden Fraktionen werden eingedampft und der Rückstand aus Diisopropylether umkristallisiert.
Ausbeute: 31,89 g (76 % d. Th.) eines farblosen, kristallinen Pulvers

| Elementaranalyse: | | | | | |
|---|---|---|---|---|---|
| ber. | C 55,05 | H 7,70 | N 8,23 | Na 2,69 | Br 9,40 |
| gef. | C 55,17 | H 7,85 | N 8,10 | Na 2,51 | Br 9,30 |

d) 10-[5-(carboxy)-1-methyl-2-oxo-3-azapentyl]-1,4,7-tris(tert.-butoxycarbonylmethyl)-1,4,7,10-tetraazacyclododecan (Natriumbromid-Komplex)

30 g (35,26 mmol) der Titelverbindung aus Beispiel 2c) werden in 500 ml Isopropanol gelöst und 3 g Palladiumkatalysator (10 % Pd/C) hinzugegeben. Man hydriert über Nacht bei Raumtemperatur. Es wird vom Katalysator abfiltriert, das Filtrat im Vakuum zur Trockne eingedampft und aus Aceton umkristallisiert.
Ausbeute: 24,41 g (91 % d. Th.) eines farblosen, kristallinen Pulvers

| Elementaranalyse: | | | | | |
|---|---|---|---|---|---|
| ber. | C 50,52 | H 7,82 | N 9,21 | Na 3,01 | Br 10,52 |
| gef. | C 50,41 | H 7,95 | N 9,10 | Na 2,91 | Br 10,37 |

e) 24-mer N-(6-DO3A-yl-5-oxo-4-azaheptanoyl)-Kaskadenpolyamid auf der Basis des N,N,N',N',N",N"-Hexakis[2-(trilysylamino)-ethyl]-trimesinsäuretriamids

6,0 g (1 mmol) des in Beispiel 1d) beschriebenen Poly-Benzyloxycarbonylamins werden in Eisessig gelöst und unter Rühren mit 33 %igem Bromwasserstoff in Eisessig versetzt. Nach 3 Stunden wird mit Diethylether die begonnene Fällung vervollständigt, das entstandene 24-Amin-hydrobromid mit Ether gewaschen und im Vakuum getrocknet.
36,52 g (48 mmol) der im vorstehenden Beispiel 2d) beschriebenen Säure werden in DMF gelöst, mit 7,35 g (48 mmol) 1-Hydroxybenzotriazol, mit 15,41 g (48 mmol) TBTU (Peboc Limited, UK) und mit 49,3 ml (288 mmol) N-Ethyldiisopropylamin versetzt und 20 Minuten bei Raumtemperatur gerührt. Diese Lösung wird anschließend mit den oben beschriebenen (1 mmol) 24-Aminhydrobromid versetzt und 4 Tage bei Raumtemperatur gerührt. Die Lösung wird im Vakuum eingeengt, das verbleibende Öl im Eisbad gekühlt und mit Trifluoressigsäure versetzt, über Nacht bei Raumtemperatur gerührt und anschließend mit Diethylether gefällt. Der Niederschlag wird im Vakuum getrocknet, in Wasser aufgenommen, auf pH 7 eingestellt, über eine YM3 Amicon® -Ultrafiltrationsmembran von niedermolekularen Anteilen gereinigt und das Retentat schließlich membranfiltriert und gefriergetrocknet.
Ausbeute: 14,4 g (85 % d.Th.)
$H_2O$-Gehalt (Karl-Fischer): 8,7 %

| Elementaranalyse (bezogen auf wasserfreie Substanz): | | | | |
|---|---|---|---|---|
| ber. | C 46,82 | H 5,98 | N 14,79 | Na 10,59 |
| gef. | C 47,04 | H 6,23 | N 14,96 | Na 10,26 |

f) 24-mer-Gd-Komplex des N-(6-DO3A-yl-5-oxo-4-azaheptanoyl)-Kaskadenpolyamids auf der Basis des N,N,N',N',N", N"-Hexakis[2-(trilysylamino)-ethyl]-trimesinsäuretriamids

8,5 g (0,5 mmol) der im vorstehenden Beispiel 2e) beschriebenen Komplexbildnersäure werden in Wasser mit verd. Salzsäure auf pH 3 gestellt, mit 2,17 g (6 mmol) $Gd_2O_3$ versetzt, 30 Minuten bei 80 °C gerührt, nach dem Abkühlen auf pH 7 eingestellt und über eine YM3 AMICON®-Ultrafiltrationsmembran entsalzt. Das Retentat wird schließlich membranfiltriert und gefriergetrocknet.
Ausbeute: 8,50 g (88 % d. Th.)
$H_2O$-Gehalt (Karl-Fischer): 7,9 %
Gd-Bestimmung (AAS): 19,4 %

| Elementaranalyse (bezogen auf wasserfreie Substanz): | | | | |
|---|---|---|---|---|
| ber. | C 41,12 | H 5,52 | N 12,99 | Gd 21,21 |
| gef. | C 40,86 | H 5,34 | N 13,25 | Gd 20,95 |

**Beispiel 3**

a) N,N'-Bis(benzyloxycarbonyl)-3-(carboxymethoxyacetyl]-3-azapentan-1,5-diamin

37,14 g (100 mmol) des in Beispiel 1a) beschriebenen Bis(benzyloxycarbonyl-aminoethyl) amins werden in DMF gelöst, im Eisbad mit 17,4 g (150 mmol) Diglykolsäureanhydrid (Janssen Chimica) und 21 ml (150 mmol) Triethylamin versetzt und anschließend über Nacht bei Raumtemperatur gerührt. Die Lösung wird im Vakuum eingedampft, der Rückstand in Ethylacetat aufgenommen und mit verdünnter Salzsäure ausgeschüttelt. Die organische Phase wird mit Natriumsulfat getrocknet und nach Filtration vom Trocknungsmittel durch Zugabe von Hexan kristallisiert.
Ausbeute: 41,4 g (85 % d. Th.)

| Elementaranalyse: | | | |
|---|---|---|---|
| ber. | C 59,13 | H 6,00 | N 8,62 |
| gef. | C 58,99 | H 5,93 | N 8,70 |

b) N,N',N'',N'''-Tetrakis{8-(Benzyloxycarbonylamino)-6-[2-(benzyloxycarbonylaminoethyl]-5-oxo-3-oxaoctanoyl}cyclen

345 mg (2 mmol) 1,4,7,10-Tetraazacyclododecan (Cyclen; Fluka) werden mit Toluol azeotrop entwässert. Zu der abgekühlten Lösung von Cyclen in Toluol wird bei Raumtemperatur eine Lösung von 4.88 g (10 mmol) N,N'-Bis (benzyloxycarbonyl)-3-[carboxymethoxyacetyl]-3-azapentan-1,5-diamin [Beispiel 3a)] in Tetrahydrofuran (THF) sowie 2,47 g (10 mmol) 2-Ethoxy-1-ethoxycarbonyl-1,2-dihydrochinolin (EEDQ; Fluka) zugegeben und über Nacht gerührt. Nach Beendigung der Reaktion wird das Produkt durch Zugabe von Hexan ausgefällt, vom Lösungsmittel abdekantiert und noch einmal aus THF/Hexan und anschließend aus THF/Toluol umgefällt. Man erhält nach Trocknung im Vakuum 2,78 g (68 % d. Th.) eines blaßgelben Feststoffs.

| Elementaranalyse: | | | |
|---|---|---|---|
| ber. | C 60,93 | H 6,29 | N 10,93 |
| gef. | C 60,68 | H 6,40 | N 10,97 |

c) Vollgeschütztes Benzyloxycarbonyl-32-Polyamin auf der Basis des aus N,N',N'',N'''-Tetrakis{8-Benzyloxycarbonylamino)-6-[2-(benzyloxycarbonylamino)-ethyl]-5-oxo-3-oxaoctanoyl}cyclen mit $N^{\alpha}$,$N^{\varepsilon}$-bis(lysyl)-Lysin("Tri-Lysin") kondensierten 32-Amins

2,05 g (1 mmol) des im Beispiel 3b) beschriebenen Okta-Benzyloxycarbonylamins werden in Eisessig gelöst und unter Rühren mit 33 igem Bromwasserstoff in Eisessig versetzt. Nach 90 Minuten wird mit Diethylether die begonnende Fällung vervollständigt, das entstandene Okta-amin-hydrobromid mit Ether gewaschen, im Vakuum getrocknet und ohne weitere Reinigung in die weiter unten beschriebene Reaktion eingesetzt.
Ausbeute: 1,6 g (quantitativ)

9,4 g (10 mmol) des in Beispiel 1c) beschriebenen geschützten "Tri-Lysins", 1,5 g (10 mmol) 1-Hydroxybenzotriazol und 3,2 g (10 mmol) 2-(1H-Benzotriazol-1-yl)-1,1,3,3-tetramethyluronium tetrafluorborat (TBTU; Peboc Limited, UK) werden in DMF gelöst und 15 Minuten gerührt. Diese Lösung wird anschließend mit 5,16 ml (30 mmol) N-Ethyldiisopropylamin und mit 1,6 g (1 mmol) des oben beschriebenen Oktaaminhydrobromids versetzt und über Nacht bei Raumtemperatur gerührt. Nach Beendigung der Reaktion wird im Vakuum eingedampft und der Rückstand mit Dichlormethan/Methanol (10:1) an Kieselgel chromatographiert.
Ausbeute: 6,0 g (72 % d. Th.)

| Elementaranalyse: | | | |
|---|---|---|---|
| ber. | C 63,32 | H 6,76 | N 10,74 |
| gef. | C 62,98 | H 6,91 | N 10,43 |

d) 32-mer N-(5-DO3A-yl-4-oxo-3-azahexanoyl)-Kaskadenpolyamid auf der Basis des im vorstehenden Beispiel 3c) beschriebenen 32-mer Amins

8,35 g (1 mmol) des in Beispiel 3c) beschriebenen 32-mer-Benzyloxycarbonylamins werden in Eisessig gelöst und unter Rühren mit 33 %igem Bromwasserstoff in Eisessig versetzt. Nach 3 Stunden wird mit Diethylether die begonnene Fällung vervollständigt, das entstandene 32-Amin-hydrobromid mit Ether gewaschen und im Vakuum getrocknet.

47,8 g (64 mmol) der im Beispiel 1h) beschriebenen Säure werden in DMF gelöst, mit 9,8 g (64 mmol) 1-Hydroxybenzotriazol, mit 20,5 g (64 mmol) TBTU (Peboc Limited, UK) und mit 65,7 ml (384 mmol) N-Ethyldiisopropylamin versetzt und 20 Minuten bei Raumtemperatur gerührt. Diese Lösung wird anschließend mit den oben beschriebenen (1 mmol)

32-Aminhydrobromid versetzt und 4 Tage bei Raumtemperatur gerührt. Die Lösung wird im Vakuum eingeengt, das verbleibende Öl im Eisbad gekühlt und mit Trifluoressigsäure versetzt, über Nacht bei Raumtemperatur gerührt und anschließend mit Diethylether gefällt. Der Niederschlag wird im Vakuum getrocknet, in Wasser aufgenommen, auf pH 7 eingestellt, über eine YM3 Amicon®-Ultrafiltrationsmembran von niedermolekularen Anteilen gereinigt und das Retentat schließlich membranfiltriert und gefriergetrocknet. Ausbeute: 17,2 g (76,4 % d.Th.)
$H_2O$-Gehalt (Karl-Fischer): 7,6 %

| Elementaranalyse (bezogen auf wasserfreie Substanz): | | | | |
|---|---|---|---|---|
| ber. | C 45,73 | H 6,12 | N 15,08 | Na 10,61 |
| gef. | C 45,89 | H 6,30 | N 14,84 | Na 10,31 |

e) 32-mer-Gd-Komplex des N-(5-DO3A-yl-4-oxo-3-azahexanoyl)-Kaskadenpolyamids auf der Basis in Beispiel 3c) beschriebenen 32-mer Amins

10,4 g (0,5 mmol) der im vorstehenden Beispiel 3d) beschriebenen Komplexbildnersäure werden in Wasser mit verd. Salzsäure auf pH 3 gestellt, mit 2,89 g (8 mmol) $Gd_2O_3$ versetzt, 30 Minuten bei 80 °C gerührt, nach dem Abkühlen auf pH 7 eingestellt und über eine YM3 AMICON®-Ultrafiltrationsmembran entsalzt. Das Retentat wird schließlich membranfiltriert und gefriergetrocknet.
Ausbeute: 12,1 g (91,1 % d. Th.)
$H_2O$-Gehalt (Karl-Fischer): 11,0 %
Gd-Bestimmung (AAS): 18,6 %

| Elementaranalyse (bezogen auf wasserfreie Substanz): | | | | |
|---|---|---|---|---|
| ber. | C 40,26 | H 5,39 | N 13,28 | Gd 21,30 |
| gef. | C 40,10 | H 5,21 | N 13,04 | Gd 21,03 |

In analoger Weise erhält man mit $Yb_2(CO_3)_3$ den Ytterbium-Komplex:

| Elementaranalyse (bezogen auf wasserfreie Substanz): | | | | |
|---|---|---|---|---|
| ber. | C 39,42 | H 5,28 | N 13,00 | Yb 22,94 |
| gef. | C 39,29 | H 5,40 | N 12,81 | Yb 22,65 |

Die nachfolgenden Beispiele 4 bis 14 dienen zur Erläuterung des Erfindungsgegenstands:

**Beispiel 4**

a) 10-[4-Carboxy-1-methyl-2-oxo-3-azabutyl]-1,4,7,10-tetraazacyclododecan-1,4,7-triessigsäure

77 g (103,1 mmol) der Titelverbindung aus Beispiel 1h werden in 500 ml Trifluoressigsäure gelöst und 3 Stunden bei Raumtemperatur gerührt. Man dampft zur Trockne ein, nimmt den Rückstand in 300 ml Wasser auf und gibt die Lösung auf eine Säule, gefüllt mit Reillex® 425 PVP. Man eluiert mit Wasser. Die produkthaltigen Fraktionen werden vereinigt und zur Trockne eingedampft, der Rückstand wird aus Methanol/Aceton umkristallisiert.
Ausbeute: 44,04 g (84 % d. Th.) eines farblosen, hygroskopischen Feststoffes
Wassergehalt: 6,5 %

| Elementaranalyse (berechnet auf wasserfreie Substanz): | | | |
|---|---|---|---|
| ber. | C 47,99 | H 6,99 | N 14,73 |
| gef. | C 47,83 | H 7,12 | N 14,55 |

b) Gadolinium-Komplex der 10-[4-Carboxy-1-methyl-2-oxo-3-azabutyl]-1,4,7,10-tetraazacyclododecan-1,4,7-triessigsäure

Zu 40 g (84,12 mmol) der Titelverbindung aus Beispiel 4a, gelöst in 400 ml Wasser, gibt man 15,27 g (42,06 mmol) Gadoliniumoxid und erwärmt 3 h auf 900C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden). Ausbeute: 50,53 g (93 % d. Th.) eines farblosen kristallinen Pulvers
Wassergehalt: 2,5 %

| Elementaranalyse (berechnet auf wasserfreie Substanz): | | | | |
|---|---|---|---|---|
| ber. | C 36,24 | H 4,80 | N 11,12 | Gd 24,97 |
| gef. | C 36,35 | H 4,95 | N 10,98 | Gd 24,80 |

**Beispiel 5**

Dysprosiumkomplex der 10-[4-Carboxy-1-methyl-2-oxo-3-azabutyl]-1,4,7,10-tetraazacyclododecan-1,4,7-triessigsäure

Zu 20 g (42,06 mmol) der Titelverbindung aus Beispiel 4a, gelöst in 200 ml Wasser, gibt man 7,84 g (21,03 mmol) Dysprosiumoxid und erwärmt 3 h auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden). Ausbeute: 24,98 (91 % d. Th.) eines farblosen kristallinen Pulvers
Wassergehalt: 2,7 %

| Elementaranalyse (berechnet auf wasserfreie Substanz): | | | | |
|---|---|---|---|---|
| ber. | C 35,94 | H 4,76 | N 11,03 | Dy 25,59 |
| gef. | C 35,85 | H 4,91 | N 10,90 | Dy 25,42 |

**Beispiel 6**

Ytterbiumkomplex der 10-[4-Carboxy-1-methyl-2-oxo-3-azabutyl]-1,4,7,10-tetraazacyclododecan-1,4,7-triessigsäure

Zu 20 g (42,06 mmol) der Titelverbindung aus Beispiel 4a, gelöst in 200 ml Wasser, gibt man 8,29 g (21,03 mmol) Ytterbiumoxid und erwärmt 3 Tage auf 90°C. Man dampft zur Trockne ein (Vakuum) und kristallisiert den Rückstandaus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden). Ausbeute: 21,79 (78 % d. Th.) eines farblosen kristallinen Pulvers
Wassergehalt: 2,8 %

| Elementaranalyse (berechnet auf wasserfreie Substanz): | | | | |
|---|---|---|---|---|
| ber. | C 35,35 | H 4,68 | N 10,85 | Yb 26,81 |
| gef. | C 35,25 | H 4,79 | N 10,68 | Yb 26,61 |

**Beispiel 7**

a) N-(2-Brombutyryl)-glycinbenzylester

Zu 100 g (296,4 mmol) Glycinbenzylester p-Toluolsulfonsäuresalz und 89,98 g (889,2 mmol) Triethylamin in 500 ml Methylenchlorid tropft man bei 0°C 65,96 g (355,7 mmol) α-Brom-Buttersäurechlorid zu. Dabei hält man die Temperatur zwischen 0°C - 5°C. Man gibt 1000 ml 5 %ige aqu. Salzsäure zu und trennt die organische Phase ab. Die organische Phase wird noch einmal mit 500 ml 5 %iger aqu. Salzsäure extrahiert, über Magnesiumsulfat getrocknet und im Vakuum zur Trockne eingedampft. Der Rückstand wird aus Di-isopropylether umkristallisiert.
Ausbeute: 75,43 g (81 % d. Th.) eines farblosen kristallinen Pulvers

| Elementaranalyse: | | | | |
|---|---|---|---|---|
| ber. | C 49,70 | H 5,13 | N 4,46 | Br 25,43 |
| gef. | C 49,51 | H 5,27 | N 4,31 | Br 25,28 |

b) 10-[4-(Benzyloxycarbonyl)-1-ethyl-2-oxo-3-azabutyl]-1,4,7,10-tetraazacyclododecan-1,4,7-triessigsäure-tri-tert-butylester

Zu 50 g (159,14 mmol) der Titelverbindung aus Beispiel 7a, 36,98 g (79,6 mmol) 1,4,7-tris(tert.butoxy-carbonylmethyl)-1,4,7,10-tetraazacyclododecan (= DO3A-tri-tert.butylester), 44 g (318,4 mmol) Kaliumcarbonat und 1 g (60 mmol) Kaliumjodid gibt man 500 ml Acetonitril und erhitzt 12 Stunden unter Rückfluß. Man filtriert von den Salzen ab und dampft das Filtrat im Vakuum zur Trockne ein. Der Rückstand wird in 800 ml Dichlormethan gelöst und 2 mal mit je 300 ml 5 %iger aqu. Natriumcarbonat-Lösung extrahiert. Die organische Phase wird über Magnesiumsulfat getrocknet und eingedampft. Nach Chromatographie an Kieselgel (Laufmittel: Dichlormethan/ Methanol= 20:1) erhält man 19,11 g der Titelverbindung (32,1 % d. Th.) als farblosen Schaum.

| Elementaranalyse: | | | |
|---|---|---|---|
| ber.: | C 62,63 | H 8,76 | N 9,36 |
| gef.: | C 62,51 | H 8,91 | N 9,18 |

c) 10-(4-Carboxy-1-ethyl-2-oxo-3-azabutyl)-1,4,7,10-tetraazacyclododecan-1,4,7-triessigsäure-tri-tert-butylester

19 g (25,40 mmol) der Titelverbindung aus Beispiel 7b löst man in 300 ml Isopropanot und gibt 2 g Palladiumkatalysator (10 % Pd/C) zu. Man hydriert über Nacht bei Raumtemperatur. Es wird vom Katalysator abfiltriert und das Filtrat zur Trockne eingedampft.
Ausbeute: 16,54 g (99 % d. Th.) eines zähflüssigen Öls

| Elementaranalyse: | | | |
|---|---|---|---|
| ber.: | C 58,43 | H 9,04 | N 10,65 |
| gef.: | C 58,65 | H 9,27 | N 10,47 |

d) 10-(4-Carboxy-1-ethyl-2-oxo-3-azabutyl)-1,4,7,10-tetraazacyclododecan-1,4,7-triessigsäure

16 g (24,32 mmol) der Titelverbindung aus Beispiel 7c werden in 100 ml Trifluoressigsäure gelöst und 3 Stunden bei Raumtemperatur gerührt. Man dampft zur Trockne ein, nimmt den Rückstand in 50 ml Wasser auf und gibt die Lösung auf eine Säule, gefüllt mit Reillex® 425 PVP. Man eluiert mit Wasser. Die produkthaltigen Fraktionen werden vereinigt und zur Trockne eingedampft, der Rückstand wird aus Methanol/Aceton umkristallisiert.
Ausbeute: 10,10 g (79 % d. Th.) eines farblosen, hygroskopischen Feststoffes
Wassergehalt: 6,9 %

| Elementaranalyse (berechnet auf wasserfreie Substanz): | | | |
|---|---|---|---|
| ber.: | C 49,07 | H 7,21 | N 14,31 |
| gef.: | C 49,28 | H 7,39 | N 14,15 |

e) Gadoliniumkomplex der 10-(4-Carboxy-1-ethyl-2-oxo-3-azabutyl)-1,4,7,10-tetraazacyclododecan-1,4,7-triessigsäure

Zu 9 g (18,38 mmol) der Titelverbindung aus Beispiel 7d, gelöst in 70 ml Wasser, gibt man 3,33 g (9,19 mmol) Gadoliniumoxid und erwärmt 3 h auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden).
Ausbeute: 11,44 g (94 % d. Th.) eines farblosen kristallinen Pulvers
Wassergehalt: 2,8 %

| Elementaranalyse (berechnet auf wasserfreie Substanz): | | | | |
|---|---|---|---|---|
| ber.: | C 37,32 | H 5,01 | N 10,88 | Gd 24,43 |
| gef.: | C 37,15 | H 5,21 | N 10,65 | Gd 24,25 |

**Beispiel 8**

Dysprosiumkomplex der 10-(4-Carboxy-1-ethyl-2-oxo-3-azabutyl)-1,4,7,10-tetraazacyclododecan-1,4,7-triessigsäure

Zu 10 g (20,43 mmol) der Titelverbindung aus Beispiel 7d gelöst in 80 ml Wasser gibt man 3,81 g (10,21 mmol) Dysprosiumoxid und erwärmt 3 h auf 90°C. Man dampft zur Trockne ein (Vakuum und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden).
Ausbeute: 12,40 g (91 % d. Th.) eines farblosen, kristallinen Pulvers
Wassergehalt: 2,7 %

| Elementaranalyse (berechnet auf wasserfreie Substanz): | | | | |
|---|---|---|---|---|
| ber.: | C 37,01 | H 4,97 | N 10,79 | Dy 25,04 |
| gef.: | C 36,85 | H 5,13 | N 10,61 | Dy 24,87 |

**Beispiel 9**

a) N-[2-Brom-2-phenyl-acetyl]-glycinsäure-tert.-butylester

Zu 50 g (296,5 mmol) Glycin-tert.-butylester Hydrochloridsalz und 90 g (889,5 mmol) Triethylamin in 500 ml Methylenchlorid tropft man bei 0°C 72,69 g (311,3 mmol) α-Brom-Phenylessigsäurechlorid zu. Dabei hält man die Temperatur zwischen 0°C - 5°C. Man gibt 1000 ml 5 %ige aqu. Salzsäure zu und trennt die organische Phase ab. Die organische Phase wird noch einmal mit 500 ml 5 %iger aqu. Salzsäure extrahiert, über Magnesiumsulfat getrocknet und im Vakuum zur Trockne eingedampft. Der Rückstand wird aus Di-isopropylether/n-Hexan umkristallisiert.
Ausbeute: 78,8 g (81 % d. Th.)

| Elementaranalyse: | | | | |
|---|---|---|---|---|
| ber.: | C 51,23 | H 5,53 | N 4,27 | Br 24,35 |
| gef.: | C 51,15 | H 5,66 | N 4,11 | Br 24,18 |

b) 1-[4-(tert.-butoxycarbonyl)-oxo-1-phenyl-3-azabutyl]-4,7,10-tris(tert.butoxycarbonylmethyl)-1,4,7,10-tetraazacyclododecan

Zu 50 g (159,14 mmol) der Titelverbindung aus Beispiel 9a, 53,12 g (114,3 mmol) 1,4,7-tris(tert.butoxy-carboxymethyl)-1,4,7,10-tetraazacyclododecan (= DO3A-tri-tert.butylester), 63,16 g (457,0 mmol) Kaliumcarbonat und 1 g (6 mmol) Kaliumjodid gibt man 500 ml Acetonitril und erhitzt 12 Stunden unter Rückfluß. Man filtriert von den Salzen ab und dampft das Filtrat im Vakuum zur Trockne ein. Der Rückstand wird in 1000 ml Dichlormethan gelöst und 2 mal mit je 400 ml 5 %iger aqu. Natriumcarbonat-Lösung extrahiert. Die vereinigten organischen Phasen werden über Magnesiumsulfat getrocknet und eingedampft. Nach Chromatographie an Kieselgel (Laufmittel: Dichlormethan/Methanol= 20:1) erhält man 27 g der Titelverbindung (31 % d. Th.) als farblosen Schaum.

| Elementaranalyse: | | | |
|---|---|---|---|
| ber. | C 63,05 | H 8,86 | N 9,19 |
| gef. | C 62,91 | H 8,98 | N 9,02 |

c) 1-(4-carboxy-2-oxo-1-phenyl-3-azabutyl)-4,7,10-tris(carboxymethyl)-1,4,7,10-tetraazacyclododecan

26 g (34,12 mmol) der Titelverbindung aus Beispiel 9b werden in 300 ml Trifluoressigsäure gelöst und 3 Stunden bei Raumtemperatur gerührt. Man dampft zur Trockne ein, nimmt den Rückstand in 80 ml Wasser auf und gibt die Lösung auf eine Säule, gefüllt mit Reillex® 425 PVP. Man eluiert mit Wasser. Die produkthaltigen Fraktionen werden vereinigt und zur Trockne eingedampft, der Rückstand wird aus Methanol/Aceton umkristallisiert.
Ausbeute: 16,22 g (81 % d. Th.) eines farblosen, hygroskopischen Feststoffes
Wassergehalt: 8,4 %

| Elementaranalyse (berechnet auf wasserfreie Substanz): | | | |
|---|---|---|---|
| ber. | C 53,62 | H 6,56 | N 13,03 |
| gef. | C 53,48 | H 6,71 | N 12,87 |

d) Gadoliniumkomplex des 1-(4-carboxy-2-oxo-1-phenyl-3-azabutyl)-4,7,10-tris(carboxymethyl)-1,4,7,10-tetraazacyclododecans

Zu 15 g (27,90 mmol) der Titelverbindung aus Beispiel 9c, gelöst in 200 ml Wasser, gibt man 5,06 g (13,95 mmol) Gadoliniumoxid und erwärmt 3 h auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden).
Ausbeute: 18,27 g (92 % d. Th.) eines farblosen kristallinen Pulvers
Wassergehalt: 2,8 %

| Elementaranalyse (berechnet auf wasserfreie Substanz): | | | | |
|---|---|---|---|---|
| ber. | C 41,67 | H 4,66 | N 10,12 | Gd 22,73 |
| gef. | C 41,40 | H 4,80 | N 9,95 | Gd 22, |

**Beispiel 10**

a) N-(2-Brompropionyl)-β-alanin

Zu 40 g (448,98 mmol) b-Alanin und 90 g (889,5 mmol) Triethylamin in 500 ml Methylenchlorid tropft man bei 0°C 72,69 g (311,3 mmol) α-Brom-Propionsäurechlorid zu. Dabei hält man die Temperatur zwischen 0°C - 5°C. Man gibt 1000 ml 5 %ige aqu. Salzsäure zu und trennt die organische Phase ab. Die organische Phase wird noch einmal mit 500 ml 5 %iger aqu. Salzsäure extrahiert, über Magnesiumsulfat getrocknet und im Vakuum zur Trockne eingedampft. Der Rückstand wird aus Aceton/Di-isopropylether umkristallisiert.
Ausbeute: 62,37 g (62 % d. Th.)

| Elementaranalyse: | | | | |
|---|---|---|---|---|
| ber. | C 32,16 | H 4,50 | N 6,25 | Br 35,66 |
| gef. | C 32,02 | H 4,65 | N 6,13 | Br 35,74 |

b) 10-(5-Carboxy-1-methyl-2-oxo-3-azapentyl)-1,4,7,10-tetraazacyclododecan-1,4,7-triessigsäure

Zu 60 g (267,80 mmol) der Titelverbindung aus Beispiel 10a, gelöst in 300 ml Acetonitril/200 ml Wasser, gibt man 46,38 g (133,9 mmol) 1,4,7-Tris(carboxymethyl)-1,4,7,10-Tetraazacyclododecan (= DO3A), 129,54 g (937,3 mmol) Kaliumcarbonat und 1 g (6 mmol) Kaliumjodid. Man erhitzt 12 Stunden unter Rückfluß. Es wird im Vakuum zur Trockne eingedampft, der Rückstand in 500 ml Methanol aufgenommen und dann von den Salzen abfiltriert. Das Filtrat wird zur Trockne eingedampft, der Rückstand in 300 ml Wasser aufgenommen und mit 5 N Salzsäure auf pH 1 gestellt. Anschließend wird über eine Säule gefüllt mit Reillex® 425 PVP gereinigt. Man eluiert mit Wasser. Die produkthaltigen Fraktionen werden im Vakuum zur Trockne eingedampft und der Rückstand aus Methanol/Aceton umkristallisiert.
Ausbeute: 19,19 g (27 % d. Th.) eines farblosen Feststoffes
Wassergehalt: 7,8 %

| Elementaranalyse (berechnet auf wasserfreie Substanz): | | | |
|---|---|---|---|
| ber. | C 49,07 | H 7,21 | N 14,31 |
| gef. | C 48,85 | H 7,31 | N 14,19 |

c) Gadoliniumkomplex der 10-(5-Carboxy-1-methyl-2-oxo-3-azapentyl)-1,4,7,10-tetraazacyclododecan-1,4,7-triessigsäure

Zu 18 g (36,77 mmol) der Titelverbindung aus Beispiel 10b gelöst in 300 ml Wasser, gibt man 6,66 g (18,38

mmol) Gadoliniumoxid und erwärmt 3 h auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden).
Ausbeute: 21,6 g (89 % d. Th.) eines farblosen kristallinen Pulvers
Wassergehalt: 2,5 %

| Elementaranalyse (berechnet auf wasserfreie Substanz): | | | | |
|---|---|---|---|---|
| ber. | C 37,32 | H 5,01 | N 10,88 | Gd 24,43 |
| gef. | C 37,15 | H 5,21 | N 10,67 | Gd 24,25 |

**Beispiel 11**

a) N-(2-Brompropionyl)-11-aminoundecansäure

Zu 30 g (149 mmol) 11-Aminoundecansäure und 45,24 g (447,1 mmol) Triethylamin in 600 ml Methylenchlorid tropft man bei 0°C 30,65 g (178,8 mmol) α-Brom-Propionsäurechlorid zu. Dabei hält man die Temperatur zwischen 0°C - 5°C. Man gibt 800 ml 5 %ige aqu. Salzsäure zu und trennt die organische Phase ab. Die organische Phase wird noch einmal mit 300 ml 5 %iger aqu. Salzsäure extrahiert, über Magnesiumsulfat getrocknet und im Vakuum zur Trockne eingedampft. Der Rückstand wird aus Aceton/ Di-isopropylether umkristallisiert.
Ausbeute: 25,55 g (51 % d. Th.)

| Elementaranalyse: | | | | |
|---|---|---|---|---|
| ber. | C 50,01 | H 7,79 | N 4,17 | Br 23,76 |
| gef. | C 49,82 | H 7,95 | N 4,03 | Br 23,59 |

b) 10-(13-Carboxy-1-methyl-2-oxo-3 -aza-tridecyl)-1,4,7,10-tetraazacyclododecan-1,4,7-triessigsäure

Zu 25 g (74,35 mmol) der Titelverbindung aus Beispiel 11a, gelöst in 250 ml Acetonitril/150 ml Wasser, gibt man 12,88 g (37,18 mmol) 1,4,7-Triscarboxymethyl-1,4,7,10-Tetraazacyclododecan (= DO3A), 35,97 g (260,3 mmol) Kaliumcarbonat und 1 g (6 mmol) Kaliumjodid. Man erhitzt 12 Stunden unter Rückfluß. Es wird im Vakuum zur Trockne eingedampft, der Rückstand in 300 ml Methanol aufgenommen und dann von den Salzen abfiltriert. Das Filtrat wird zur Trockne eingedampft, der Rückstand in 300 ml Wasser aufgenommen und mit 5 N Salzsäure auf pH 1 gestellt. Anschließend wird über eine Säule gefüllt mit Reillex® 425 PVP gereinigt. Man eluiert mit Wasser. Die produkthaltigen Fraktionen werden im Vakuum zur Trockne eingedampft und der Rückstand aus Methanol/Aceton umkristallisiert.
Ausbeute: 6,63 g (27 % d. Th.) eines farblosen Feststoffes
Wassergehalt: 8,9 %

| Elementaranalyse (berechnet auf wasserfreie Substanz): | | | |
|---|---|---|---|
| ber. | C 55,89 | H 8,54 | N 11,64 |
| gef. | C 55,71 | H 8,70 | N 11,57 |

c) Gadoliniumkomplex der 10-(13-Carboxy-1-methyl-2-oxo-3-aza-tridecyl)-1,4,7,10-tetraazacyclododecan-1,4,7-triessigsäure

Zu 6 g (9,97 mmol) der Titelverbindung aus Beispiel 11b, gelöst in 80 ml Wasser, gibt man 1,81 g (10,21 mmol) Gadoliniumoxid und erwärmt 3 h auf 90°C. Man dampft zur Trockne ein (Vakuum und kristallisiert den Rückstand aus 90 % aqu. 2-Propanol um. Die Kristalle werden abgesaugt, einmal mit Ethanol, dann mit Aceton und zum Schluß mit Diethylether gewaschen und im Vakuumofen bei 130°C getrocknet (24 Stunden).
Ausbeute: 6,75 g (87 % d. Th.) eines farblosen, kristallinen Pulvers
Wassergehalt: 2,9 %

| Elementaranalyse (berechnet auf wasserfreie Substanz): | | | | |
|---|---|---|---|---|
| ber. | C 44,49 | H 6,40 | N 9,26 | Gd 20,80 |
| gef. | C 44,28 | H 6,55 | N 9,11 | Gd 20,63 |

**Beispiel 12**

a) N-(2-Brompropionyl)-alanin

Zu 30 g (336,7 mmol) Alanin und 102,2 g (1010,2 mmol) Triethylamin in 600 ml Methylenchlorid tropft man bei 0°C 69,26 g (404 mmol) α-Brom-Propionsäurechlorid zu. Dabei hält man die Temperatur zwischen 0°C - 5°C. Man gibt 1000 ml 5 %ige aqu. Salzsäure zu und trennt die organische Phase ab. Die organische Phase wird noch einmal mit 400 ml 5 %iger aqu. Salzsäure extrahiert, über Magnesiumsulfat getrocknet und im Vakuum zur Trockne eingedampft. Der Rückstand wird aus Aceton/ Di-isopropylether umkristallisiert.
Ausbeute: 52,05 g (69 % d. Th.)

| Elementaranalyse (berechnet auf wasserfreie Substanz): | | | | |
|---|---|---|---|---|
| ber. | C 32,16 | H 4,50 | N 6,25 | Br 35,66 |
| gef. | C 32,33 | H 4,70 | N 6,13 | Br 35,41 |

b) 10-(4-Carboxy-1-methyl-2-oxo-3-azapentyl)-1,4,7,10-tetraazacyclododecan-1,4,7-triessigsäure

Zu 50 g (223,2 mmol) der Titelverbindung aus Beispiel 12a, gelöst in 300 ml Acetonitril/200 ml Wasser, gibt man 38,65 g (111,6 mmol) 1,4,7-Triscarboxymethyl-1,4,7,10-Tetraazacyclododecan (= DO3A), 108 g (781,2 mmol) Kaliumcarbonat und 1 g (6 mmol) Kaliumjodid. Man erhitzt 12 Stunden unter Rückfluß. Es wird im Vakuum zur Trockne eingedampft, der Rückstand in 500 ml Methanol aufgenommen und dann von den Salzen abfiltriert. Das Filtrat wird zur Trockne eingedampft, der Rückstand in 300 ml Wasser aufgenommen und mit 5 N Salzsäure auf pH 1 gestellt. Anschließend wird über eine Säule gefüllt mit Reillex® 425 PVP gereinigt. Man eluiert mit Wasser. Die produkthaltigen Fraktionen werden im Vakuum zur Trockne eingedampft und der Rückstand aus Methanol/Aceton umkristallisiert.
Ausbeute: 17,72 g (30 % d. Th.) eines farblosen Feststoffes
Wassergehalt: 7,5 %

| Elementaranalyse (berechnet auf wasserfreie Substanz): | | | |
|---|---|---|---|
| ber. | C 49,07 | H 7,21 | N 14,31 |
| gef. | C 49,23 | H 7,38 | N 14,15 |

c) Gadoliniumkomplex der 10-(4-Carboxy-1-methyl-2-oxo-3-azapentyl)-1,4,7,10-tetraazacyclododecan-1,4,7-triessigsäure

Zu 15 g (30,64 mmol) der Titelverbindung aus Beispiel 12b, gelöst in 150 ml Wasser, gibt man 5,55 g (15,32 mmol) Gadoliniumoxid und erwärmt 3 h auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden). Ausbeute: 18,22 g (90 % d. Th.) eines farblosen, kristallinen Pulvers
Wassergehalt: 2,6 %

| Elementaranalyse (berechnet auf wasserfreie Substanz): | | | | |
|---|---|---|---|---|
| ber. | C 37,32 | H 5,01 | N 10,88 | Gd 24,43 |
| gef. | C37,13 | H 5,20 | N 10,61 | Gd 24,41 |

**Beispiel 13**

a) N-(2-Brompropionyl)-valin

Zu 40 g (341,4 mmol) Valin und 103,7 g (1024 mmol) Triethylamin in 600 ml Methylenchlorid tropft man bei 0°C 70,2 g (409,7 mmol) α-Brom-Propionsäurechlorid zu. Dabei hält man die Temperatur zwischen 0°C - 5°C. Man gibt 1000 ml 5 %ige aqu. Salzsäure zu und trennt die organische Phase ab. Die organische Phase wird noch einmal mit 500 ml 5 %iger aqu. Salzsäure extrahiert, über Magnesiumsulfat getrocknet und im Vakuum zur Trockne eingedampft. Der Rückstand wird aus Aceton/ Di-isopropylether umkristallisiert.
Ausbeute: 59,39 g (69 % d. Th.)

| Elementaranalyse (berechnet auf wasserfreie Substanz): | | | | |
|---|---|---|---|---|
| ber. | C 38,11 | H 5,60 | N 5,56 | Br 31,69 |
| gef. | C 38,01 | H 5,75 | N 5,41 | Br 31,48 |

b) 10-(4-Carboxy-1,5-dimethyl-2-oxo-3-azahexyl]-1,4,7,10-tetraazacyclododecan-1,4,7-triessigsäure

Zu 55 g (218,2 mmol) der Titelverbindung aus Beispiel 13a, gelöst in 200 ml Acetonitril/200 ml Wasser, gibt man 37,8 g (109,7 mmol) 1,4,7-Triscarboxymethyl-1,4,7,10-Tetraazacyclododecan (= DO3A), 106,13 g (767,9 mmol) Kaliumcarbonat und 1 g (6 mmol) Kaliumjodid. Man erhitzt 12 Stunden unter Rückfluß. Es wird im Vakuum zur Trockne eingedampft, der Rückstand in 500 ml Methanol aufgenommen und dann von den Salzen abfiltriert. Das Filtrat wird zur Trockne eingedampft, der Rückstand in 300 ml Wasser aufgenommen und mit 5 N Salzsäure auf pH 1 gestellt. Anschließend wird über eine Säule gefüllt mit Reillex® 425 PVP gereinigt. Man eluiert mit Wasser. Die produkthaltigen Fraktionen werden im Vakuum zur Trockne eingedampft und der Rückstand aus Methanol/Aceton umkristallisiert.
Ausbeute: 17,57 g (29 % d. Th.) eines farblosen Feststoffes
Wassergehalt: 6,3 %

| Elementaranalyse (berechnet auf wasserfreie Substanz): | | | |
|---|---|---|---|
| ber. | C 51,05 | H 7,59 | N 13,53 |
| gef. | C 51,18 | H 7,70 | N 13,39 |

c) Gadoliniumkomplex der 10-(4-Carboxy-1,5-dimethyl-2-oxo-3-azahexyl]-1,4,7,10-tetraazacyclododecan-1,4,7-triessigsäure

Zu 15 g (28,98 mmol) der Titelverbindung aus Beispiel 13b, gelöst in 150 ml Wasser, gibt man 5,25 g (14,49 mmol) Gadoliniumoxid und erwärmt 3 h auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden).
Ausbeute: 18,57 g (93 % d. Th.) eines farblosen, kristallinen Pulvers
Wassergehalt: 2,5 %

| Elementaranalyse (berechnet auf wasserfreie Substanz): | | | | |
|---|---|---|---|---|
| ber. | C 39,33 | H 5,40 | N 10,42 | Gd 23,41 |
| gef. | C 39,17 | H 5,55 | N 10,31 | Gd 23,27 |

**Beispiel 14**

a) N-(2-Bromacetyl)-glycin-tert.-butylester

Zu 50 g (296,5 mmol) Glycin-tert.-butylester Hydrochloridsalz und 90 g (889,5 mmol) Triethylamin in 500 ml Methylenchlorid tropft man bei 0°C 77,8 g (385,5 mmol) α-Brom-Essigsäurebromid zu. Dabei hält man die Temperatur zwischen 0°C - 5°C. Man gibt 1000 ml 5 %ige aqu. Salzsäure zu und trennt die organische Phase ab. Die organische Phase wird noch einmal mit 500 ml 5 %iger aqu. Salzsäure extrahiert, über Magnesiumsulfat getrocknet und im Vakuum zur Trockne eingedampft. Der Rückstand wird aus Diisopropylether/n-Hexan umkristallisiert.
Ausbeute: 30,5 g (61 % d. Th.)

| Elementaranalyse: | | | | |
|---|---|---|---|---|
| ber. | C 38,11 | H 5,60 | N 5,56 | Br 31,69 |
| gef. | C 37,92 | H 5,76 | N 5,38 | Br 31,42 |

b) 10-[4-(tert.Butoxycarbonyl)-2-oxo-3-azabutyl]-1,4,7,10-tetraazacyclododecan-1,4,7-triessigsäure-tri-tert.-butylester

Zu 20,35 g (80,70 mmol) der Titelverbindung aus Beispiel 14a, 25 g (53,8 mmol) 1,4,7-Tris(tert.butoxy-carboxymethyl)-1,4,7,10-tetraazacyclododecan (= DO3A-tri-tert.butylester), 29,74 g (215,8 mmol) Kaliumcarbonat und 1 g (6 mmol) Kaliumjodid gibt man 200 ml Acetonitril und erhitzt 12 Stunden unter Rückfluß Man filtriert von den Salzen ab und dampft das Filtrat im Vakuum zur Trockne ein. Der Rückstand wird in 800 ml Dichlormethan gelöst und 2 mal mit 200 ml 5 %iger aqu. Natriumcarbonat-Lösung extrahiert. Die organische Phase wird über Magnesiumsulfat getrocknet und eingedampft. Nach Chromatographie an Kieselgel (Laufmittel: Dichlormethan/ Methanol= 20:1) erhält man 25,09 g der Titelverbindung (68 % d. Th.) als farblosen Schaum.

| Elementaranalyse: | | | |
|---|---|---|---|
| ber. | C 59,54 | H 9,26 | N 10,21 |
| gef. | C 59,35 | H 9,42 | N 10,03 |

c) 10-[4-Carboxy-2-oxo-3-azabutyl]-1,4,7,10-tetraazacyclododecan-1,4,7-triessigsäure

25 g (36,45 mmol) der Titelverbindung aus Beispiel 14b werden in 300 ml Trifluoressigsäure gelöst und 3 Stunden bei Raumtemperatur gerührt. Man dampft zur Trockne ein, nimmt den Rückstand in 80 ml Wasser auf und gibt die Lösung auf eine Säule, gefüllt mit Reillex® 425 PVP. Man eluiert mit Wasser. Die produkthaltigen Fraktionen werden vereinigt und zur Trockne eingedampft, der Rückstand wird aus Methanol/Aceton umkristallisiert.
Ausbeute: 15,24 g (84 % d. Th.) eines farblosen, hygroskopischen Feststoffes Wassergehalt: 7,3 %

| Elementaranalyse (berechnet auf wasserfreie Substanz): | | | |
|---|---|---|---|
| ber. | C 46,85 | H 6,77 | N 15,18 |
| gef. | C 46,61 | H 6,95 | N 15,02 |

d) Gadoliniumkomplex der 10-[4-Carboxy-2-oxo-3-azabutyl]-1,4,7,10-tetraazacyclododecan-1,4,7-triessigsäure

Zu 15 g (32,50 mmol) der Titelverbindung aus Beispiel 14c, gelöst in 200 ml Wasser, gibt man 5,86 g (16,25 mmol) Gadoliniumoxid und erwärmt 3 h auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden).
Ausbeute: 18,92 g (92 % d. Th.) eines farblosen, kristallinen Pulvers
Wassergehalt: 2,7 %

| Elementaranalyse (berechnet auf wasserfreie Substanz): | | | | |
|---|---|---|---|---|
| ber. | C 35,11 | H 4,58 | N 11,37 | Gd 25,54 |
| gef. | C 34,92 | H 4,71 | N 11,14 | Gd 25,33 |

Die nachfolgenden Beispiele dienen zur Erläuterung der Verwendung der erfindungsgemäßen makrocyclischen Metallkomplexcarbonsäuren:

**Beispiel 15**

24-mer-Gd-Komplex des N-(5-DO3A-yl-4-oxo-3-azahexanoyl)-Kaskadenpolyamids auf der Basis des N,N,N',N',N",N"-Hexakis[2-(trilysylamino)-ethyl]-trimesinsäuretriamids

4,2 g (0,7 mmol) des im Beispiel 1d beschriebenen vollgeschützten Benzyloxycarbonyl-24mer-Polyamins auf der Basis des N,N,N',N',N",N"-Hexakis[2-(trilysylamino)-ethyl]-trimesinsäuretriamids werden in Eisessig gelöst und unter Rühren mit 33 %igem Bromwasserstoff in Eisessig versetzt. Nach 3 Stunden wird mit Diethylether die begonnene Fällung vervollständigt, das entstandene 24mer-Amin-hydrobromid mit Ether gewaschen, im Vakuum getrocknet (3,3 g, quantitativ) und ohne weitere Reinigung in die folgende Reaktion eingesetzt.
31,74 g (50,4 mmol, dreifacher Überschuß) des in Beispiel 4b beschriebenen Gd-Komplexes der 10-[4-Carboxy-1-methyl-2-oxo-3-azabutyl]-1,4,7,10-tetraazacyclododecan-1,4,7-triessigsäure werden in 250 ml Formamid in der Wärme gelöst. Nach Abkühlen auf Raumtemperatur werden 13,69 g (55,4 mmol) 2-Ethoxy-1-ethoxycarbonyl-1,2-dihydrochinolin (EEDQ, Fluka), 3,3 g (0,7 mmol) des oben beschriebenen Tetracosahydrobromids und 1,70 g (16,8 mmol) Triethylamin zugesetzt und über Nacht bei Raumtemperatur gerührt. Die Lösung wird anschließend mit Aceton versetzt, der Niederschlag abgesaugt, getrocknet, in Wasser aufgenommen, von unlöslichen Anteilen abfiltriert und

das Filtrat über eine Amicon® YM3 Ultrafiltrationsmembran (cut off 3.000 Da) entsalzt bzw. von niedermolekularen Bestandteilen gereinigt. Das Retentat wird anschließend gefriergetrocknet.
Ausbeute: 10,46 g (78 % d. Th.)
$H_2O$-Gehalt (Karl-Fischer): 9 %
Gd-Bestimmung (AAS): 18,8 %

| Elementaranalyse (berechnet auf wasserfreie Substanz): | | | | |
|---|---|---|---|---|
| ber. | C 40,26 | H 5,35 | N 13,24 | Gd 21,62 |
| gef. | C 40,07 | H 5,32 | N 13,14 | Gd 21,43 |

Das MALDI-MS (Matrix Assisted Laser Desorption/Ionization Mass Spectrometry: z.B.: F. Hillenkamp, M. Karas, R. Beavis, B.T. Chait, Anal. Chem. 63, 1193A (1991)) zeigt Signale bei m/z= ca. 17,470 (24mer), ca. 16,960 (23mer) und ca. 16,480 (22mer) und belegt damit eine geringere Nebenproduktverteilung als das nach Beispiel 1 k erhaltene Produkt, das folgende Verteilung aufweist: Signale bei m/2 = ca. 17,450 (24mer), ca. 16,830 (23mer), ca. 16,230 (22mer), ca. 15,680 (21mer), ca. 15070 (20mer) und 14.450 (19mer).

**Beispiel 16**

24mer-Gd-Komplex des N-(6-DO3A-yl-5-oxo-4-azaheptanoyl)-Kaskadenpolyamids auf der Basis des N,N,N',N',N",N"-Hexakis[2-(trilysylamino)-ethyl]-trimesinsäuretriamids

4,2 g (0,7 mmol) des in Beispiel 1d beschriebenen vollgeschützten Benzyloxycarbonyl-24mer-Polyamins auf der Basis des N,N,N',N',N",N"-Hexakls[2-(trilysylamino)-ethyl]-trimesinsäuretriamids werden in Eisessig gelöst und unter Rühren mit 33 %igem Bromwasserstoff in Eisessig versetzt. Nach 3 Stunden wird mit Diethylether die begonnene Fällung vervollständigt, das entstandene 24mer-Amin-hydrobromid mit Ether gewaschen, im Vakuum getrocknet (3,3 g, quantitativ) und ohne weitere Reinigung in die folgende Reaktion eingesetzt.
32,45 g (50,4 mmol, dreifacher Überschuß) des in Beispiel 10c beschriebenen Gd-Komplexes der 10-(5-Carboxy-1-methyl-2-oxo-3-aza-pentyl)-1,4,7,10-tetraazacyclododecan-1,4,7-triessigsäure werden in 250 ml Formamid in der Wärme gelöst. Nach Abkühlen aufRaumtemperatur werden 13,69 g (55,4 mmol) 2-Ethoxy-1-ethoxycarbonyl-1,2-dihydrochinolin (EEDQ, Fluka), 3,3 g (0,7 mmol) des oben beschriebenen Tetracosahydrobromids und 1,70 g (16,8 mmol) Triethylamin zugesetzt und über Nacht bei Raumtemperatur gerührt. Die Lösung wird anschließend mit Aceton versetzt, der Niederschlag abgesaugt, getrocknet, in Wasser aufgenommen, von unlöslichen Anteilen abfiltriert und das Filtrat über eine Amicon® YM3 Ultrafiltrationsmembran (cut off 3.000 Da) entsalzt bzw. von niedermolekularen Bestandteilen gereinigt. Das Retentat wird anschließend gefriergetrocknet.
Ausbeute: 10,53 g (77 % d. Th.)
$H_2O$-Gehalt (Karl-Fischer): 9 %
Gd-Bestimmung (AAS): 18,5 %

| Elementaranalyse (berechnet auf wasserfreie Substanz): | | | | |
|---|---|---|---|---|
| ber. | C 41,12 | H 5,52 | N 12,99 | Gd 21,21 |
| gef. | C 40,95 | H 5,62 | N 12,78 | Gd 21,01 |

Das MALDI-MS zeigt Signale bei m/z = ca. 17,790 (24mer), ca. 17,180 (23mer) und ca. 16,540 (22mer).

**Beispiel 17**

32-mer-Dysprosium-Komplex des N-(5-DO3A-yl-4-oxo-3-azahexanoyl)-Kaskadenpolyamids auf der Basis des in Beispiel 3c) beschriebenen 32mer Amins

8,35 g (1 mmol) des in Beispiel 3c) beschriebenen 32-mer-Benzyloxycarbonylamins werden in Eisessig gelöst und unter Rühren mit 33 %igem Bromwasserstoff in Eisessig versetzt. Nach 3 Stunden wird mit Diethylether die begonnene Fällung vervollständigt, das entstandene 32-Amin-hydrobromid mit Ether gewaschen, im Vakuum getrocknet (quantitative Ausbeute) und ohne weitere Reinigung in die folgende Reaktion eingesetzt. 60,96 g (96 mmol, dreifacher Überschuß) des in Beispiel 5 beschriebenen Dy-Komplexes der 10-(4-Carboxy-1-methyl-2-oxo-3-azabutyl)-1,4,7,10-tetraazacyclododecan-1,4,7-triessigsäure werden in 500 ml Formamid in der Wärme gelöst. Nach Abkühlen aufRaumtemperatur werden 26,1 g (105,6 mmol) 2-Ethoxy-1-ethoxycarbonyl-1,2-dihydrochinolin (EEDQ, Fluka), 1

mmol des oben beschriebenen Dotriaconta-hydrobromids und 3,24 g (32 mmol) Triethylamin zugesetzt und über Nacht bei Raumtemperatur gerührt. Die Lösung wird anschließend mit Aceton versetzt, der Niederschlag abgesaugt, getrocknet, in Wasser aufgenommen, von unlöslichen Anteilen abfiltriert und das Filtrat über eine Amicon® YM3 Ultrafiltrationsmembran (cut off 3.000 Da) entsalzt bzw. von niedermolekularen Bestandteilen gereinigt. Das Retentat wird anschließend gefriergetrocknet.
Ausbeute: 19,0 g (75 % d. Th.)
$H_2O$-Gehalt (Karl-Fischer): 6 %
Dy-Bestimmung (AAS): 19,7 %

| Elementaranalyse (berechnet auf wasserfreie Substanz): | | | | |
|---|---|---|---|---|
| ber. | C 39,98 | H 5,35 | N 13,19 | Dy 21,85 |
| gef. | C 39,83 | H 5,26 | N 13,28 | Dy 21,51 |

Das MALDI-MS zeigt Signale bei m/z = ca. 23,800 (32mer), ca. 23,200 (31mer) und ca. 22,600 (30mer).

**Beispiel für einen in-vivo Vergleich mit einem extrazellulären Kontrastmittel**

Die Eignung der im Beispiel 1k) beschriebenen Verbindung als blood-pool-agent wird im folgenden Versuch gezeigt.

Als Versuchstiere dienen fünf 300 - 350 g schwere männliche (Schering-SPF-)Ratten. Vor dem Versuch wird das Abdomen eröffnet, der Darm verlagert und dann durch das hintere Bauchfell hindurch mit einer chirurgischen Nadel die Nierengefäße (arteriell+venös) beider Seiten abgebunden. Anschließend wird die Bauchhöhle wieder verschlossen. Danach werden je Tier 0.3 ml (jeweils 50 mmol/L) der folgenden Kontrastmittel-Lösung intravenös appliziert: Gemisch aus je 1 Teil der Verbindung aus Beispiel 1k), im folgenden Verbindung 1 genannt, und dem Dysprosium-Komplex des 10-(1-Hydroxymethyl-2,3-dihydroxypropyl)-1,4,7-tris(carboxymethyl)-1,4,7,10-tetraazacyclododecans, hergestellt analog der Vorschrift in der Europ. Patentanmeldung EP 448 191, im folgenden Verbindung 2 genannt. Über einen Katheter in der Arteria carotis communis werden Blutproben zu folgenden Zeitpunkten entnommen: 15, 30, 45, 60, 90 sec, 3, 5, 10, 15 min p.i. In den gewonnenen Blutproben werden jeweils parallel die Konzentrationen an Gadolinium (Gd) und Dysprosium (Dy) mittels Atomemissionsspektrometrie (ICP-AES) gemessen. Der im Blutraum verbliebene Anteil der injizierten Kontrastmittel Verbindung 1 (Gd) und Verbindung 2 (Dy, Vergleichssubstanz) kann durch die unterschiedliche Markierung im gleichen Tier verglichen werden. Da eine renale Ausscheidung nicht möglich ist, kann der Abfall der Blutkonzentration nur auf eine Verteilung in den Bluträumen und auf die Diffusion in das interstitielle Gewebe zurückzuführen sein.

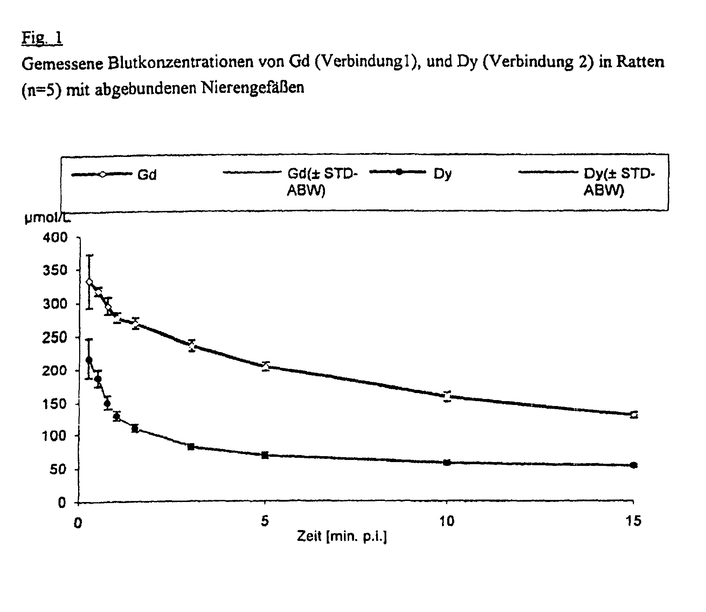

Ergebnisse: Die Diffusion von Verbindung 1 in das Interstitium ist im Vergleich zu einem extrazellulären Kontrastmittel Verbindung 2 deutlich verlangsamt (siehe Figur 1).

Das extrazelluläre Kontrastmittel (Verbindung 2) diffundiert so schnell in die interstitiellen Räume des Körpers, daß bereits nach 3-5 Minuten p.i. ein Equilibrium erreicht wird (angezeigt durch konstanten Blutspiegel). Im Gegensatz dazu werden beim Kaskadenpolymer (Verbindung 1) nicht nur stets höhere Blutkonzentrationen gemessen (Hinweis auf kleineres Verteilungsvolumen), sondern es wird auch über den gesamten Untersuchungszeitraum von 15 Minuten noch kein Equilibrium erreicht (Hinweis auf nur sehr langsam verlaufende Diffusion ins interstitielle Gewebe). Das bedeutet, daß sich Verbindung 1 als Blutpool-Kontrastmittel verhält.

**Beispiel für eine Lymphknotenanreicherung am Meerschweinchen**

Die unter Beispiel 1k genannte erfindungsgemäße Verbindung wurde 30 min bis 24 h nach subkutaner Gabe (10 µmol Gadolinium/kg Körpergewicht, Hinterpfote s.c.) an stimulierten Meerschweinchen (komplettes Freund-Adjuvant; jeweils 0,1 ml i.m. in den rechten und linken Ober- und Unterschenkel; 2 Wochen vor Gabe der Prüfsubstanzen) hinsichtlich ihrer Lymphknotenanreicherung in drei aufeinanderfolgenden Lymphknotenstationen (popliteal, inguinal, iliakal) untersucht. Hierbei wurden die nachfolgend aufgelisteten Ergebnisse (Ermittlung der Gadolinium-Konzentration mittels ICP-AES) erhalten:

| Zeitpunkt der Lymphknotenentnahme | Gadolinium-Konzentration in drei aufeinanderfolgenden Lymphknotenstationen [µmol/l] [% Dosis/g Gewebe] | | | |
|---|---|---|---|---|
| | Popliteal | Inguinal | Iliakal | Verhältnis |
| 30 min p.i. | 921 µmol/l<br>20,1 % | 387 µmol/l<br>8,5 % | 215 µmol/l<br>4,7 % | 10 : 4,2 : 2,3 |
| 90 min p.i. | 659 µmol/l<br>14,4 % | 120 µmol/l<br>2,6 % | 68 µmol/l<br>1,5 % | 10: 1,8 : 1,0 |
| 4 h p.i. | 176 µmol/l<br>3,9 % | 79 µmol/l<br>1,7 % | 47 µmol/l<br>1,0 % | 10 : 4,5 : 2,7 |
| 24 h p.i. | 62 µmol/l<br>1,4 % | 13 µmol/l<br>0.3 % | 28 µmol/l<br>0,6 % | 10 : 2,1 : 4,5 |

**Patentansprüche**

1. Verbindungen der allgemeinen Formel II

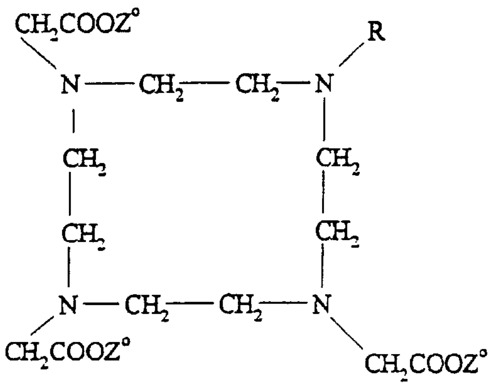

(II),

wobei

Zo für ein Metallionenäquivalent der Ordnungszahlen 58-71 und
R für eine $CHX^1$-CO-NH-$CHY^1$-$(CH_2)_f$-COOH-Gruppe steht, worin $X^1$ und $Y^1$ unabhängig voneinander einen geradkettigen oder verzweigten $C_1$-$C_7$-Alkylrest, eine Phenyl- oder Benzylgruppe und $Y^1$ zusätzlich ein Wasserstoffatom und f die Ziffern 0 bis 9 bedeuten.

2. Verbindungen gemäß Anspruch 1, **dadurch gekennzeichnet, daß** Z° für ein Metallionenäquivalent der Ordnungszahlen 64, 66 und 70 steht.

3. Verbindungen gemäß Anspruch 1, **dadurch gekennzeichnet, daß** $X^1$ für eine Methylgruppe steht.

4. Verbindungen gemäß Anspruch 1, **dadurch gekennzeichnet, daß** $Y^1$ für ein Wasserstoffatom steht.

5. Verbindungen gemäß Anspruch 1, **dadurch gekennzeichnet, daß** f für die Ziffern 0,1 oder 2 steht.

6. Verbindung gemäß Anspruch 1:

Gadolinium-Komplex der 10-[4-Carboxy-1-methyl-2-oxo-3-azabutyl]-1,4,7,10-tetraazacyclododecan-1,4,7-triessigsäure.

7. Verfahren zur Herstellung von Verbindungen der allgemeinen Formel II gemäß Anspruch 1,

**dadurch gekennzeichnet, daß** man Verbindungen der allgemeinen Formel III

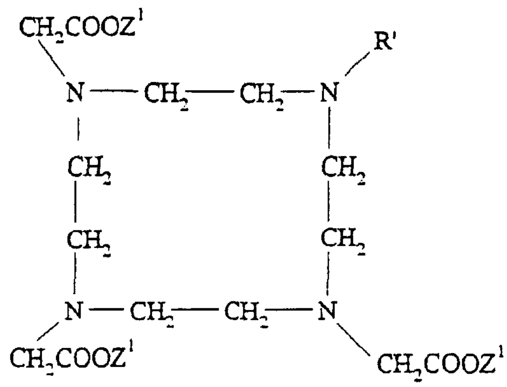

(III),

worin

R' die Bedeutung von R hat, wobei die darin enthaltene Carboxylgruppe gegebenenfalls in geschützter Form vorliegt, und

$Z^1$ für ein Wasserstoffatom oder eine Carboxylschutzgruppe steht, nach Abspaltung der gegebenenfalls vorhandenen Carboxylschutzgruppen, in an sich bekannter Weise mit einem Metalloxid oder Metallsalz eines Elements der Ordnungszahlen 58-71 umsetzt.

**8.** Verwendung von Verbindungen gemäß Anspruch 1 zur Hestellung von Mitteln für die NMR- oder Röntgendiagnostik.

## Claims

**1.** Compounds of general formula II

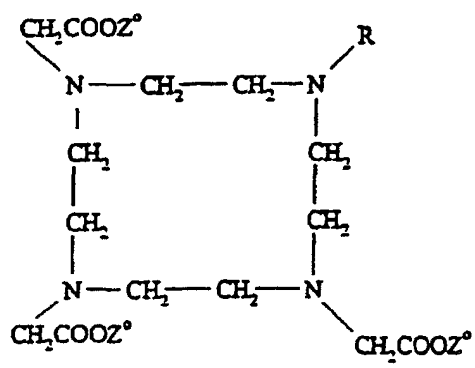

(II),

where

Z° stands for a metal ion equivalent of atomic numbers 58-71 and

R stands for a $CHX^1$-CO-NH-$CHY^1$-$(CH_2)_f$-COOH group, in which

$X^1$ and $Y^1$, independently of one another, mean a hydrogen atom, a straight-chain or branched $C_1$-$C_7$ alkyl radical, a phenyl or benzyl group and $Y^1$ additionally means a hydrogen atom and

f means numbers 0 to 9.

2. Compounds according to Claim 1, **characterized in that** Z° stands for a metal ion equivalent of atomic numbers 64, 66 and 70.

3. Compounds according to Claim 1, **characterized in that** $X^1$ stands for a methyl group.

4. Compounds according to Claim 1, **characterized in that** $Y^1$ stands for a hydrogen atom.

5. Compounds according to Claim 1, **characterized in that** f stands for numbers 0, 1 or 2.

6. Compound according to Claim 1:

   Gadolinium complex of 10-[4-carboxy-1-methyl-2-oxo-3-azabutyl]-1,4,7,10-tetraazacyclododecane-1,4,7-triacetic acid.

7. Process for the production of compounds of general formula II according to Claim 1, **characterized in that** compounds of general formula III

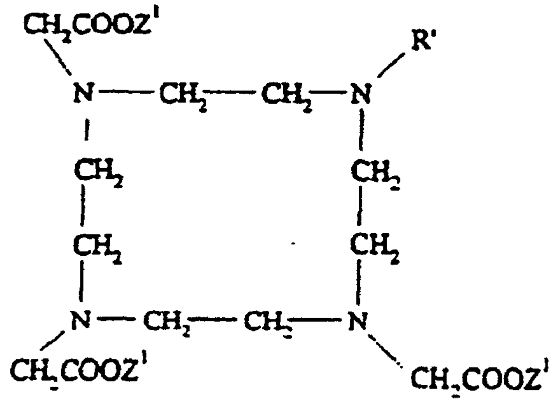

(III)

in which

R' has the meaning of R, where the carboxyl group contained therein is optionally present in protected form and

$Z^1$ stands for a hydrogen atom or a carboxyl protective group, after cleavage of the optionally present carboxyl protective groups, are reacted in a way known in the art with a metal oxide or metal salt of an element of atomic numbers 58-71.

8. Use of compounds according to Claim 1 for producing agents for NMR diagnosis or diagnostic radiology.

## Revendications

1. Composés de formule générale II

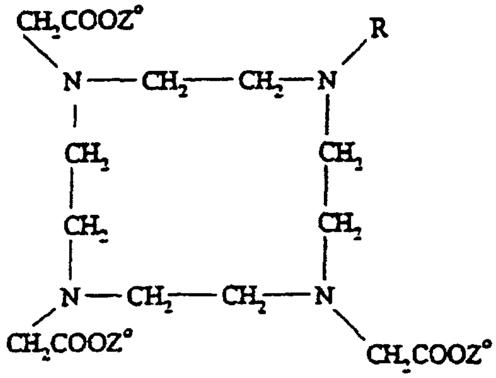

(II),

dans laquelle

Z représente un équivalent d'ion métallique des numéros atomiques 58 à 71 et
R représente un groupe $CHX^1$-CO-NH-$CHY^1$-$(CH_2)_f$-COOH dans lequel $X^1$ et $Y^1$ représentent indépendamment l'un de l'autre un groupe alkyle en $C_1$ à C7, à chaîne linéaire ou ramifiée, un groupe phényle ou benzyle et $Y^1$ représente de plus un atome d'hydrogène, f représentant un nombre de 0 à 9.

2. Composés selon la revendication 1, **caractérisés en ce que** Z° représente un équivalent d'ion métallique des numéros atomiques 64, 66 et 70.

3. Composés selon la revendication 1, **caractérisés en ce que** $X^1$ représente un groupe méthyle.

4. Composés selon la revendication 1, **caractérisés en ce que** $Y^1$ représente un atome d'hydrogène.

5. Composés selon la revendication 1, **caractérisés en ce que** f représente les nombres 0, 1 ou 2.

6. Composé selon la revendication 1 qui est le complexe de gadolinium de l'acide 10-[4-carboxy-1-méthyl-2-oxo-3-azabutyl]-1,4,7,10-tétraazacyclododécane-1,4,7-triacétique.

7. Procédé pour la préparation de composés de formule générale II selon la revendication 1, **caractérisé en ce que** l'on fait réagir de manière connue en soi des composés de formule générale III

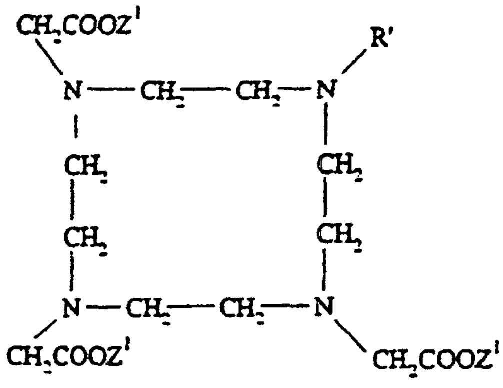

(III),

dans laquelle

R' a la même signification que R, le groupe carboxyle qui y est contenu étant éventuellement présent sous forme protégée et
$Z^1$ représente un atome d'hydrogène ou un groupe carboxyle de protection, après dissociation des groupes carboxyle de protection éventuellement présents, avec un oxyde métallique ou un sel métallique d'un élément

des numéros atomiques 58 à 71.

**8.** Utilisation de composés selon la revendication 1 pour la préparation d'agents de diagnostic par RMN ou par rayons X.

Fig. 1

Gemessene Blutkonzentrationen von Gd (Verbindung1), und Dy (Verbindung 2) in Ratten (n=5) mit abgebundenen Nierengefäßen